(19) Europäisches Patentamt
European Patent Office
Office européen des brevets

(11) **EP 4 803 151 A2**

(12) **EUROPEAN PATENT APPLICATION**

(43) Date of publication:
**09.09.2026 Bulletin 2026/37**

(21) Application number: **26193892.2**

(22) Date of filing: **20.09.2024**

(51) International Patent Classification (IPC):
***A61P 35/00*** *(2006.01)*

(52) Cooperative Patent Classification (CPC):
**A61P 35/00; C07K 16/28;** C07K 2317/24; C07K 2317/33; C07K 2317/565; C07K 2317/567; C07K 2317/92

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority: **21.09.2023 PCT/CN2023/120494**
**11.03.2024 PCT/CN2024/081042**

(62) Document number(s) of the earlier application(s) in accordance with Art. 76 EPC:
**24867537.3 / 4 780 854**

(71) Applicant: **Lepu Biopharma Co., Ltd.**
**Shanghai 201112 (CN)**

(72) Inventors:
- **CAI, Zhijian**
  **Shanghai 201112 (CN)**
- **WU, Xin**
  **Shanghai 201112 (CN)**
- **CUI, Feifei**
  **Shanghai 201112 (CN)**
- **FANG, Lei**
  **Shanghai 201112 (CN)**

(74) Representative: **Gleiss Große Schrell und Partner mbB**
**Leitzstraße 45**
**70469 Stuttgart (DE)**

Remarks:
•The complete document including Reference Table(s) and the Sequence Listing(s) can be downloaded from the EPO website
•This application was filed on 23.07.2026 as a divisional application to the application mentioned under INID code 62.

(54) **ANTI-DLL3 ANTIBODIES AND USES THEREOF**

(57) This disclosure provides anti-DLL3 antibodies, including murine antibodies, humanized antibodies, and those with further optimized CDR sequences. The newly disclosed antibodies have excellent binding specificity and improved activities as compared to other anti-DLL3 antibodies under development.

EP 4 803 151 A2

## Description

## BACKGROUND

Lung cancer is the malignant tumor with the highest mortality rate in the world, and small cell lung cancer (SCLC) accounts for about 15% among them. Unlike other types of cancer, SCLC is highly invasive and can undergo metastasis in early stages. The clinical outcomes include frequent SCLC recurrences and drug resistance. In addition, the overall survival (OS) rate of SCLC is not high due to a lack of targeted therapy and challenges for early detection. Although immunotherapy is available for first-line treatment of extensive stage small cell lung cancer (ES-SCLC), such as with PD-L1 monoclonal antibody (Durvalumab)-coupled chemotherapy, the OS has not improved significantly. Therefore, there is an urgent need for improved therapies for SCLC.

Delta-like ligand 3 (DLL3) is an inhibitory Notch ligand in the Notch ligand family. In contrast to other members of the family, such as DLL1 and DLL4, the human DLL3 protein has a distinct structure that consists of one Delta/Serrate/LAG-2 (DSL) domain, six epithelial growth factor (EGF) like repeats, a 21 amino acid (aa) trans-membrane domain (TM), and a 105 aa intracellular domain (ICD).

DLL3 is highly expressed on the cell surface in SCLC tumors and other tumor types of neuroendocrine origin, including glioblastoma multiforme (GBM), large cell neuroendocrine lung tumors (LCNEC), metastatic melanoma, small cell bladder cancer (SCBC), and neuroendocrine prostate cancer (NEPC). The DLL3 protein is expressed by more than 80% of SCLC tumors overall, with a high degree of uniformity across neoplastic cells. In contrast, only minimal expression is observed in normal tissues (e.g., neurons, pancreatic islet cells, and pituitary cells), and it is exclusively cytoplasmic. In preclinical models, DLL3 expression promotes SCLC cell migration and invasion through a mechanism that involves the control of epithelial mesenchymal transition protein Snail.

The distinct expression pattern of DLL3 in SCLC and other neuroendocrine tumors has enabled the development of therapeutics that use DLL3 to specifically target these tumor types. Currently, multiple active clinical studies in SCLC and other neuroendocrine tumors are evaluating these DLL3-specific drugs. Several types of modalities targeting DLL3, including bispecific Ab (BsAb), chimeric antigen receptor T cell therapy (CAR-T) and antibody drug conjugation (ADC) are currently under clinical investigation.

Tarlatamab (AMG757), a half-life extended bi-specific T-cell engager (BiTE) molecule targeting DLL3 and CD3 simultaneously leading to T-cell-mediated tumor lysis, exhibited manageable safety with encouraging response durability in patients with relapsed/refractory SCLC in the phase I study DeLLphi-300 (NCT03319940). The objective response rate (ORR) was 23.4% (95% CI, 15.7 to 32.5) including two complete and 23 partial responses. The median duration of response (DoR) was 12.3 months (95% CI, 6.6 to 14.9). The disease control rate (DCR) was 51.4% (95% CI, 41.5 to 61.2). The median progression-free survival (PFS) and overall survival (OS) were 3.7 months (95% CI, 2.1 to 5.4) and 13.2 months (95% CI, 10.5 to not reached), respectively.

Other DLL3-based T cell engagers, including BI764532 from Boehringer-Ingelheim and HPN328 from Harpoon Therapeutics, also showed promising anti-tumor efficacy in their respective phase I clinical studies. Rovalpituzumab tesirine (Rova-T) is an ADC containing a DLL3-targeting antibody rovalpituzumab tethered to a DNA cross-linking agent pyrrolobenzodiazepine (PDB) by means of a protease-cleavable linker. In the Phase 3 TAHOE study comparing Rova-T with topotecan as second-line therapy in DLL3-high advanced or metastatic SCLC, Rova-T exhibited an inferior OS and higher toxicity rates. AMG 119, the first CAR-T cell therapy for SCLC, was associated with a manageable safety profile and promising anti-tumor activity in 5 adult subjects in its phase I study although the enrollment is currently paused. LB2102, an autologous CAR-T cell therapy, was recently approved by the FDA for phase I clinical development for the treatment of adult patients with ES-SCLC. Overall, exploring different drug types targeting DLL3 will hopefully bring more choices for SCLC treatments.

## SUMMARY

The present disclosure, in various embodiments, provides antibodies and antigen-binding fragments specific to the human DLL3 protein. Experimental testing shows that these newly identified antibodies can bind to the human DLL3 protein potently and specifically, without interacting with the DLL1 and DLL4 variants. These antibodies also cross-react with the cynomolgus DLL3 protein facilitating preclinical studies.

One embodiment of the present disclosure provides an antibody or antigen-binding fragment thereof which has specificity to the human delta-like ligand 3 (DLL3) protein and comprises a heavy chain variable region (VH) comprising a VH CDR1, a VH CDR2 and a VH CDR3, and a light chain variable region (VL) comprising a VL CDR1, a VL CDR2, and a VL CDR3, wherein: (a) the VH CDR1 comprises the amino acid sequence of SEQ ID NO: 37; the VH CDR2 comprises an amino acid sequence selected from the group consisting of SEQ ID NO: 38 and 111-117; the VH CDR3 comprises the amino acid sequence of SEQ ID NO: 39; the VL CDR1 comprises the amino acid sequence of SEQ ID NO: 40; the VL CDR2 comprises the amino acid sequence of SEQ ID NO: 41; and the VL CDR3 comprises the amino acid sequence of SEQ ID

NO: 42, (b) the VH CDR1, VH CDR2, VH CDR3, VL CDR1, VL CDR2 and VL CDR3 comprise, respectively, the amino acid sequences of SEQ ID NO: 13-18; (c) the VH CDR1, VH CDR2, VH CDR3, VL CDR1, VL CDR2 and VL CDR3 comprise, respectively, the amino acid sequences of SEQ ID NO: 19-24; (d) the VH CDR1, VH CDR2, VH CDR3, VL CDR1, VL CDR2 and VL CDR3 comprise, respectively, the amino acid sequences of SEQ ID NO: 25-30; (e) the VH CDR1, VH CDR2, VH CDR3, VL CDR1, VL CDR2 and VL CDR3 comprise, respectively, the amino acid sequences of SEQ ID NO: 31-36; or (f) the VH CDR1, VH CDR2, VH CDR3, VL CDR1, VL CDR2 and VL CDR3 comprise, respectively, the amino acid sequences of SEQ ID NO: 43-48.

In some embodiments, (a) the VH CDR1 comprises the amino acid sequence of SEQ ID NO: 37; the VH CDR2 comprises an amino acid sequence selected from the group consisting of SEQ ID NO: 38 and 111-117; the VH CDR3 comprises the amino acid sequence of SEQ ID NO: 39; the VL CDR1 comprises the amino acid sequence of SEQ ID NO: 40; the VL CDR2 comprises the amino acid sequence of SEQ ID NO: 41; and the VL CDR3 comprises the amino acid sequence of SEQ ID NO: 42.

In some embodiments, the VH comprises an amino acid sequence selected from the group consisting of SEQ ID NO: 65-69 and 104-110 or a peptide having at least 90% sequence identity to an amino acid sequence selected from the group consisting of SEQ ID NO: 65-69 and 104-110. In some embodiments, the VL comprises an amino acid sequence selected from the group consisting of SEQ ID NO: 70-73 or a peptide having at least 90% sequence identity to an amino acid sequence selected from the group consisting of SEQ ID NO: 70-73. In some embodiments, the VH comprises an amino acid sequence selected from the group consisting of SEQ ID NO: 104-110 and the VL comprises the amino acid sequence of SEQ ID NO: 73.

In some embodiments, (b) the VH CDR1, VH CDR2, VH CDR3, VL CDR1, VL CDR2 and VL CDR3 comprise, respectively, the amino acid sequences of SEQ ID NO: 13-18.

In some embodiments, the VH comprises the amino acid sequence of SEQ ID NO: 1 or a peptide having at least 90% sequence identity to SEQ ID NO:1, and the VL comprises the amino acid sequence of 2 or a peptide having at least 90% sequence identity to SEQ ID NO:2.

In some embodiments, the antibody or antigen-binding fragment thereof comprises a heavy chain comprising the amino acid sequence of SEQ ID NO:49, and a light chain comprising the amino acid sequence of SEQ ID NO:50.

In some embodiments, (c) the VH CDR1, VH CDR2, VH CDR3, VL CDR1, VL CDR2 and VL CDR3 comprise, respectively, the amino acid sequences of SEQ ID NO: 19-24.

In some embodiments, the VH comprises the amino acid sequence of SEQ ID NO: 3 or a peptide having at least 90% sequence identity to SEQ ID NO:3, and the VL comprises the amino acid sequence of 4 or a peptide having at least 90% sequence identity to SEQ ID NO:4.

In some embodiments, the antibody or antigen-binding fragment thereof comprises a heavy chain comprising the amino acid sequence of SEQ ID NO:51, and a light chain comprising the amino acid sequence of SEQ ID NO:52.

In some embodiments, wherein (d) the VH CDR1, VH CDR2, VH CDR3, VL CDR1, VL CDR2 and VL CDR3 comprise, respectively, the amino acid sequences of SEQ ID NO: 25-30.

In some embodiments, the VH comprises the amino acid sequence of SEQ ID NO: 5 or a peptide having at least 90% sequence identity to SEQ ID NO:5, and the VL comprises the amino acid sequence of 6 or a peptide having at least 90% sequence identity to SEQ ID NO:6.

In some embodiments, the antibody or antigen-binding fragment thereof comprises a heavy chain comprising the amino acid sequence of SEQ ID NO:53, and a light chain comprising the amino acid sequence of SEQ ID NO:54.

In some embodiments, (e) the VH CDR1, VH CDR2, VH CDR3, VL CDR1, VL CDR2 and VL CDR3 comprise, respectively, the amino acid sequences of SEQ ID NO: 31-36.

In some embodiments, the VH comprises the amino acid sequence of SEQ ID NO: 7 or a peptide having at least 90% sequence identity to SEQ ID NO:7, and the VL comprises the amino acid sequence of 8 or a peptide having at least 90% sequence identity to SEQ ID NO:8.

In some embodiments, the antibody or antigen-binding fragment thereof comprises a heavy chain comprising the amino acid sequence of SEQ ID NO:55, and a light chain comprising the amino acid sequence of SEQ ID NO:56.

In some embodiments, (f) the VH CDR1, VH CDR2, VH CDR3, VL CDR1, VL CDR2 and VL CDR3 comprise, respectively, the amino acid sequences of SEQ ID NO: 43-48.

In some embodiments, the VH comprises the amino acid sequence of SEQ ID NO: 11 or a peptide having at least 90% sequence identity to SEQ ID NO: 11, and the VL comprises the amino acid sequence of 12 or a peptide having at least 90% sequence identity to SEQ ID NO:12.

In some embodiments, the antibody or antigen-binding fragment thereof comprises a heavy chain comprising the amino acid sequence of SEQ ID NO:59, and a light chain comprising the amino acid sequence of SEQ ID NO:60.

Also provided, in one embodiment, is an antibody or antigen-binding fragment thereof which has specificity to the human delta-like ligand 3 (DLL3) protein, which competes with the antibody or fragment thereof of the present disclosure in binding to the DLL3 protein.

Also provided, in one embodiment, is an antibody or antigen-binding fragment thereof which has specificity to the

human delta-like ligand 3 (DLL3) protein, which binds to the EGF3-4 domain or the EGF6 domain.

Also provided is a multispecific antibody comprising an antigen-binding fragment of the present disclosure and one or more antibody or antigen-binding fragment having binding specificity to a target antigen that is not DLL3.

Yet another embodiment provides a chimeric antigen receptor (CAR) comprising an antigen-binding fragment of the present disclosure, a transmembrane domain, a costimulatory domain, and a CD3ξ intracellular domain.

Methods and uses for treating cancer are also provided. In some embodiments, the cancer is selected from the group consisting of ovarian cancer, prostate cancer, cancer of the urinary tract, pancreatic cancer, lung cancer, breast cancer, bladder cancer, colorectal cancer, endometrial cancer, esophageal cancer, head and neck cancer, kidney cancer, leukemia, liver cancer, lymphoma, melanoma, and thyroid cancer. In some embodiments, the cancer is small cell lung cancer (SCLC).

## BRIEF DESCRIPTION OF THE DRAWINGS

**FIG. 1A-1C** show ELISA binding of anti-DLL3 antibodies to human/cynomolgus/mouse DLL3 protein with his tag.

**FIG. 2A and 2B** show ELISA binding of anti-DLL3 antibodies to human paralogues DLL1 and DLL4.

**FIG. 3** shows the epitope domain mapping results for anti-DLL3 antibodies.

**FIG. 4A and 4B** show cell-based binding of anti-DLL3 antibodies to HEK293 cell lines expressing human or cynomolgus DLL3.

**FIG. 5A and 5B** show cell-based binding of anti-DLL3 antibodies to tumor cell lines, including SHP77 and NCI-H82.

**FIG. 6A and 6B** show cell-based binding of anti-DLL3 antibodies to human paralogues DLL1 and DLL4 expressed on CHO-K1 cells.

**FIG. 7** shows ELISA binding of anti-DLL3 humanized antibodies to human DLL3 protein with his tag.

**FIG. 8A and 8B** show cell-based binding of anti-DLL3 humanized antibodies to HEK293 cell lines expressing human or cynomolgus DLL3.

**FIG. 9A and 9B** show cell-based binding of anti-DLL3 humanized antibodies to tumor cell lines, including SHP77 and NCI-H82.

**FIG. 10A and 10B** show cell-based binding of anti-DLL3 humanized antibodies to human paralogues DLL1 and DLL4 expressed on CHO-K1 cells.

**FIG. 11A and 11B** show cell-based binding of anti-DLL3 humanized and PTM-removed antibodies to HEK293 cells expressing human DLL3 and SHP77 cells.

## DETAILED DESCRIPTION

### *Definitions*

It is to be noted that the term "a" or "an" entity refers to one or more of that entity; for example, "an antibody," is understood to represent one or more antibodies. As such, the terms "a" (or "an"), "one or more," and "at least one" can be used interchangeably herein.

As used herein, an "antibody" or "antigen-binding moiety" refers to a polypeptide or a polypeptide complex that specifically recognizes and binds to an antigen. An antibody can be a whole antibody and any antigen binding fragment or a single chain thereof. Thus the term "antibody" includes any protein or peptide containing molecule that comprises at least a portion of an immunoglobulin molecule having biological activity of binding to the antigen. Examples of such include, but are not limited to a complementarity determining region (CDR) of a heavy or light chain or a ligand binding portion thereof, a heavy chain or light chain variable region, a heavy chain or light chain constant region, a framework (FR) region, or any portion thereof, or at least one portion of a binding protein.

A full-length antibody comprises two heavy chains and two light chains. The variable regions of the light and

heavy chains are responsible for antigen binding. The variable domains of the heavy chain and light chain may be referred to as "VH" and "VL", respectively. The variable regions in both chains generally contain three highly variable loops called the complementarity determining regions (CDRs) (light chain (LC) CDRs including LC-CDR1, LC-CDR2, and LC-CDR3, heavy chain (HC) CDRs including HC-CDR1, HC-CDR2, and HC-CDR3). CDR boundaries for the antibodies and antigen-binding fragments disclosed herein may be defined or identified by the conventions of Kabat, Chothia, or Al-Lazikani (Al-Lazikani 1997; Chothia 1985; Chothia 1987; Chothia 1989; Kabat 1987; Kabat 1991). The three CDRs of the heavy or light chains are interposed between flanking stretches known as framework regions (FRs), which are more highly conserved than the CDRs and form a scaffold to support the hypervariable loops. The constant regions of the heavy and light chains are not involved in antigen binding, but exhibit various effector functions. Antibodies are assigned to classes based on the amino acid sequence of the constant region of their heavy chain. The five major classes or isotypes of antibodies are IgA, IgD, IgE, IgG, and IgM, which are characterized by the presence of $\alpha$, $\delta$, $\varepsilon$, $\gamma$, and $\mu$ heavy chains, respectively. Several of the major antibody classes are divided into subclasses such as IgG1 ($\gamma$1 heavy chain), IgG2 ($\gamma$2 heavy chain), IgG3 ($\gamma$3 heavy chain), IgG4 ($\gamma$4 heavy chain), IgA1 ($\alpha$1 heavy chain), or IgA2 ($\alpha$2 heavy chain).

The term "half antibody" as used herein refers to one immunoglobulin heavy chain associated with one immunoglobulin light chain. One skilled in the art will readily appreciate that a half-antibody may encompass a fragment thereof and may also have an antigen binding domain consisting of a single variable domain, *e.g.*, originating from a camelidae.

The term "single chain half antibody" as used herein refers to a single chain polypeptide comprising a VL domain, optionally a CL domain, a tether, a VH domain, optionally a CH1 domain, a hinge domain, a CH2 domain and a CH3 domain, wherein said domains are positioned relative to each other in an N-terminal to C-terminal direction as follows: VL-tether-VH-hinge-CH2-CH3, VL-tether-VH-partial hinge-CH2-CH3, VL-tether-VH- hinge variant -CH2-CH3, or VL-CL-tether-VH-CH1-hinge-CH2-CH3.

The expression "single domain antibodies" (sdAbs) or "single variable domain (SVD) antibodies" generally refers to antibodies in which a single variable domain (VH or VL) can confer antigen binding. In other words, the single variable domain does not need to interact with another variable domain in order to recognize the target antigen. Examples of single domain antibodies include those derived from camelids (lamas and camels) and cartilaginous fish (*e.g.*, nurse sharks) and those derived from recombinant methods from humans and mouse antibodies (Nature (1989) 341:544-546; Dev Comp Immunol (2006) 30:43-56; Trend Biochem Sci (2001) 26:230-235; Trends Biotechnol (2003):21:484-490; WO 2005/035572; WO 03/035694; Febs Lett (1994) 339:285-290; WO00/29004; WO 02/051870). When the sdAb contains only a heavy chain, it can be exchangeably used with "VHH" or "single heavy chain variable domain antibody" or "nanobody".

The terms "antibody fragment" or "antigen-binding fragment", as used herein, is a portion of an antibody such as $F(ab')_2$, $F(ab)_2$, Fab', Fab, Fv, scFv and the like. Regardless of structure, an antibody fragment binds with the same antigen that is recognized by the intact antibody. The term "antibody fragment" includes aptamers, spiegelmers, and diabodies. The term "antibody fragment" also includes any synthetic or genetically engineered protein that acts like an antibody by binding to a specific antigen to form a complex.

A "Fab" with regard to an antibody refers to a monovalent antigen-binding fragment of the antibody consisting of a single light chain (both variable and constant regions) bound to the variable region and first constant region of a single heavy chain by a disulfide bond. Fab can be obtained by papain digestion of an antibody at the residues proximal to the N-terminus of the disulfide bond between the heavy chains of the hinge region.

A "Fab'" refers to a Fab fragment that includes a portion of the hinge region, which can be obtained by pepsin digestion of an antibody at the residues proximal to the C-terminus of the disulfide bond between the heavy chains of the hinge region and thus is different from Fab in a small number of residues (including one or more cysteines) in the hinge region.

A "$F(ab)_2$" refers to a dimer of Fab' that comprises two light chains and part of the two heavy chains.

A "single-chain variable fragment" or "scFv" refers to a fusion protein of the variable regions of the heavy ($V_H$) and light chains ($V_L$) of immunoglobulins. In some aspects, the regions are connected with a short linker peptide of ten to about 25 amino acids. The linker can be rich in glycine for flexibility, as well as serine or threonine for solubility, and can either connect the N-terminus of the $V_H$ with the C-terminus of the $V_L$, or vice versa. This protein retains the specificity of the original immunoglobulin, despite removal of the constant regions and the introduction of the linker. ScFv molecules are known in the art and are described, *e.g.*, in US patent 5,892,019.

The term antibody encompasses various broad classes of polypeptides that can be distinguished biochemically. Those skilled in the art will appreciate that heavy chains are classified as gamma, mu, alpha, delta, or epsilon (y, $\mu$, $\alpha$, $\delta$, $\varepsilon$) with some subclasses among them (*e.g.*, $\gamma$ 1- $\gamma$4). It is the nature of this chain that determines the "class" of the antibody as IgG, IgM, IgA IgG, or IgE, respectively. The immunoglobulin subclasses (isotypes) *e.g.*, $IgG_1$, $IgG_2$, $IgG_3$, $IgG_4$, $IgG_5$, etc. are well characterized and are known to confer functional specialization. Modified versions of each of these classes and isotypes are readily discernable to the skilled artisan in view of the instant disclosure and, accordingly, are within the scope of the instant disclosure. All immunoglobulin classes are clearly within the scope of the present disclosure, the following

discussion will generally be directed to the IgG class of immunoglobulin molecules. With regard to IgG, a standard immunoglobulin molecule comprises two identical light chain polypeptides of molecular weight approximately 23,000 Daltons, and two identical heavy chain polypeptides of molecular weight 53,000-70,000. The four chains are typically joined by disulfide bonds in a "Y" configuration wherein the light chains bracket the heavy chains starting at the mouth of the "Y" and continuing through the variable region.

Antibodies, antigen-binding moieties, variants, or derivatives thereof of the disclosure include, but are not limited to, polyclonal, monoclonal, multispecific, human, humanized, primatized, or chimeric antibodies, single chain antibodies, epitope-binding fragments, *e.g.*, Fab, Fab' and $F(ab')_2$, Fd, Fvs, single-chain Fvs (scFv), single-chain antibodies, disulfide-linked Fvs (sdFv), fragments comprising either a VK or VH domain, fragments produced by a Fab expression library, and anti- idiotypic (anti-Id) antibodies (including, *e.g.*, anti-Id antibodies to LIGHT antibodies disclosed herein). Immunoglobulin or antibody molecules of the disclosure can be of any type (*e.g.*, IgG, IgE, IgM, IgD, IgA, and IgY), class (*e.g.*, IgG1, IgG2, IgG3, IgG4, IgAl and IgA2) or subclass of immunoglobulin molecule.

Light chains are classified as either kappa or lambda (κ, λ). Each heavy chain class may be bound with either a kappa or lambda light chain. In general, the light and heavy chains are covalently bonded to each other, and the "tail" portions of the two heavy chains are bonded to each other by covalent disulfide linkages or non-covalent linkages when the immunoglobulins are generated either by hybridomas, B cells or genetically engineered host cells. In the heavy chain, the amino acid sequences run from an N-terminus at the forked ends of the Y configuration to the C-terminus at the bottom of each chain.

Both the light and heavy chains are divided into regions of structural and functional homology. The terms "constant" and "variable" are used functionally. In this regard, it will be appreciated that the variable domains of both the light (VK) and heavy (VH) chain portions determine antigen recognition and specificity. Conversely, the constant domains of the light chain (CK) and the heavy chain (CH1, CH2 or CH3) confer important biological properties such as secretion, transplacental mobility, Fc receptor binding, complement binding, and the like. By convention the numbering of the constant region domains increases as they become more distal from the antigen-binding site or amino- terminus of the antibody. The N-terminal portion is a variable region and at the C-terminal portion is a constant region; the CH3 and CK domains actually comprise the carboxy-terminus of the heavy and light chain, respectively.

As indicated above, the variable region allows the antibody to selectively recognize and specifically bind epitopes on antigens. That is, the VK domain and VH domain, or subset of the complementarity determining regions (CDRs), of an antibody combine to form the variable region that defines a three-dimensional antigen-binding site. This quaternary antibody structure forms the antigen-binding site present at the end of each arm of the Y. More specifically, the antigen-binding site is defined by three CDRs on each of the VH and VK chains (*i.e.* CDR-H1, CDR-H2, CDR-H3, CDR-L1, CDR-L2 and CDR-L3). In some instances, *e.g.*, certain immunoglobulin molecules derived from camelid species or engineered based on camelid immunoglobulins, a complete immunoglobulin molecule may consist of heavy chains only, with no light chains. *See*, *e.g.*, Hamers-Casterman et al., Nature 363:446-448 (1993).

In naturally occurring antibodies, the six "complementarity determining regions" or "CDRs" present in each antigen-binding domain are short, non-contiguous sequences of amino acids that are specifically positioned to form the antigen-binding domain as the antibody assumes its three-dimensional configuration in an aqueous environment. The remainder of the amino acids in the antigen-binding domains, referred to as "framework" regions, show less inter-molecular variability. The framework regions largely adopt a β-sheet conformation and the CDRs form loops which connect, and in some cases form part of, the β -sheet structure. Thus, framework regions act to form a scaffold that provides for positioning the CDRs in correct orientation by inter-chain, non-covalent interactions. The antigen-binding domain formed by the positioned CDRs defines a surface complementary to the epitope on the immunoreactive antigen. This complementary surface promotes the non-covalent binding of the antibody to its cognate epitope. The amino acids comprising the CDRs and the framework regions, respectively, can be readily identified for any given heavy or light chain variable region by one of ordinary skill in the art, since they have been precisely defined (*see* "Sequences of Proteins of Immunological Interest," Kabat, E., et al., U.S. Department of Health and Human Services, (1983); and Chothia and Lesk, J. Mol. Biol., 196:901-917 (1987)).

In the case where there are two or more definitions of a term which is used and/or accepted within the art, the definition of the term as used herein is intended to include all such meanings unless explicitly stated to the contrary. A specific example is the use of the term "complementarity determining region" ("CDR") to describe the non-contiguous antigen combining sites found within the variable region of both heavy and light chain polypeptides. This particular region has been described by Kabat et al., U.S. Dept. of Health and Human Services, "Sequences of Proteins of Immunological Interest" (1983) and by Chothia et al., J. Mol. Biol. 196:901-917 (1987), which are incorporated herein by reference in their entireties. The CDR definitions according to Kabat and Chothia include overlapping or subsets of amino acid residues when compared against each other. Nevertheless, application of either definition to refer to a CDR of an antibody or variants thereof is intended to be within the scope of the term as defined and used herein. The appropriate amino acid residues which encompass the CDRs as defined by each of the above cited references are set forth in the table below as a comparison. The exact residue numbers which encompass a particular CDR will vary depending on the sequence and size

of the CDR. Those skilled in the art can routinely determine which residues comprise a particular CDR given the variable region amino acid sequence of the antibody.

| | Kabat | Chothia |
|---|---|---|
| CDR-H1 | 31-35 | 26-32 |
| CDR-H2 | 50-65 | 52-58 |
| CDR-H3 | 95-102 | 95-102 |
| CDR-L1 | 24-34 | 26-32 |
| CDR-L2 | 50-56 | 50-52 |
| CDR-L3 | 89-97 | 91-96 |

Kabat *et al.* also defined a numbering system for variable domain sequences that is applicable to any antibody. One of ordinary skill in the art can unambiguously assign this system of "Kabat numbering" to any variable domain sequence, without reliance on any experimental data beyond the sequence itself. As used herein, "Kabat numbering" refers to the numbering system set forth by Kabat et al., U.S. Dept. of Health and Human Services, "Sequence of Proteins of Immunological Interest" (1983).

In addition to table above, the Kabat number system describes the CDR regions as follows: CDR-H1 begins at approximately amino acid 31 (*i.e.*, approximately 9 residues after the first cysteine residue), includes approximately 5-7 amino acids, and ends at the next tryptophan residue. CDR-H2 begins at the fifteenth residue after the end of CDR-H1, includes approximately 16-19 amino acids, and ends at the next arginine or lysine residue. CDR-H3 begins at approximately the thirty third amino acid residue after the end of CDR-H2; includes 3-25 amino acids; and ends at the sequence W-G-X-G, where X is any amino acid. CDR-L1 begins at approximately residue 24 (*i.e.*, following a cysteine residue); includes approximately 10-17 residues; and ends at the next tryptophan residue. CDR-L2 begins at approximately the sixteenth residue after the end of CDR-L1 and includes approximately 7 residues. CDR-L3 begins at approximately the thirty third residue after the end of CDR-L2 (*i.e.*, following a cysteine residue); includes approximately 7-11 residues and ends at the sequence F or W-G-X-G, where X is any amino acid.

Antibodies disclosed herein may be from any animal origin including birds and mammals. Preferably, the antibodies are human, murine, donkey, rabbit, goat, guinea pig, camel, llama, horse, or chicken antibodies. In another embodiment, the variable region may be condricthoid in origin (*e.g.*, from sharks).

As used herein, the term "heavy chain constant region" includes amino acid sequences derived from an immunoglobulin heavy chain. A polypeptide comprising a heavy chain constant region comprises at least one of: a CH1 domain, a hinge (*e.g.*, upper, middle, and/or lower hinge region) domain, a CH2 domain, a CH3 domain, or a variant or fragment thereof. For example, an antigen-binding polypeptide for use in the disclosure may comprise a polypeptide chain comprising a CH1 domain; a polypeptide chain comprising a CH1 domain, at least a portion of a hinge domain, and a CH2 domain; a polypeptide chain comprising a CH1 domain and a CH3 domain; a polypeptide chain comprising a CH1 domain, at least a portion of a hinge domain, and a CH3 domain, or a polypeptide chain comprising a CH1 domain, at least a portion of a hinge domain, a CH2 domain, and a CH3 domain. In another embodiment, a polypeptide of the disclosure comprises a polypeptide chain comprising a CH3 domain. Further, an antibody for use in the disclosure may lack at least a portion of a CH2 domain (*e.g.*, all or part of a CH2 domain). As set forth above, it will be understood by one of ordinary skill in the art that the heavy chain constant region may be modified such that they vary in amino acid sequence from the naturally occurring immunoglobulin molecule.

The heavy chain constant region of an antibody disclosed herein may be derived from different immunoglobulin molecules. For example, a heavy chain constant region of a polypeptide may comprise a CH1 domain derived from an $IgG_1$ molecule and a hinge region derived from an $IgG_3$ molecule. In another example, a heavy chain constant region can comprise a hinge region derived, in part, from an $IgG_1$ molecule and, in part, from an $IgG_3$ molecule. In another example, a heavy chain portion can comprise a chimeric hinge derived, in part, from an $IgG_1$ molecule and, in part, from an $IgG_4$ molecule.

As used herein, the term "light chain constant region" includes amino acid sequences derived from antibody light chain. Preferably, the light chain constant region comprises at least one of a constant kappa domain or constant lambda domain.

A "light chain-heavy chain pair" refers to the collection of a light chain and heavy chain that can form a dimer through a disulfide bond between the CL domain of the light chain and the CH1 domain of the heavy chain.

As previously indicated, the subunit structures and three-dimensional configuration of the constant regions of the various immunoglobulin classes are well known. As used herein, the term "VH domain" includes the amino terminal

variable domain of an immunoglobulin heavy chain and the term "CH1 domain" includes the first (most amino terminal) constant region domain of an immunoglobulin heavy chain. The CH1 domain is adjacent to the VH domain and is amino terminal to the hinge region of an immunoglobulin heavy chain molecule.

The "CH1 domain" (also referred to as "C1" of "H1" domain) usually extends from about amino acid 118 to about amino acid 215 (EU numbering system).

As used herein, the term "hinge region" includes the portion of a heavy chain molecule that joins the CH1 domain to the CH2 domain, a region in IgG corresponding to Glu216 to Pro230 of human IgG1, EU numbering system (Burton, Molec. Immunol.22:161-206 (1985)). Hinge regions of other IgG isotypes may be aligned with the IgG1 sequence by placing the first and last cysteine residues forming inter-heavy chain S-S bonds in the same positions. This hinge region is flexible, thus allowing the two N-terminal antigen-binding regions to move independently. Hinge regions can be subdivided into three distinct domains: upper, middle, and lower hinge domains (Roux et al., J. Immunol 161:4083 (1998)).

As used herein the term "CH2 domain" includes the portion of a heavy chain molecule that extends, *e.g.*, from about residue 244 to residue 360 of an antibody using conventional numbering schemes (residues 244 to 360, Kabat numbering system; and residues 231-340, EU numbering system; *see* Kabat et al., U.S. Dept. of Health and Human Services, "Sequences of Proteins of Immunological Interest" (1983). The CH2 domain is unique in that it is not closely paired with another domain. Rather, two N-linked branched carbohydrate chains are interposed between the two CH2 domains of an intact native IgG molecule. It is also well documented that the CH3 domain extends from the CH2 domain to the C-terminal of the IgG molecule and comprises approximately 108 residues.

The "CH3 domain" (also referred to as "C3" domain) comprises the stretch of residues C-terminal to a CH2 domain in an Fc region (*i.e.* from about amino acid residue 341 to the C-terminal end of an antibody sequence, typically at amino acid residue 446 or 447 of an IgG, EU numbering system).

The term "Fc region", "Fc domain" or "fragment crystallizable region" herein is used to define a C-terminal region of an immunoglobulin heavy chain, including native-sequence Fc regions and variant Fc regions. Although the boundaries of the Fc region of an immunoglobulin heavy chain might vary, the human IgG heavy-chain Fc region is usually defined to stretch from an amino acid residue at position Cys226, or from Pro230, to the carboxyl-terminus thereof. The C-terminal lysine (residue 447 according to the EU numbering system) of the Fc region may be removed, for example, during production or purification of the antibody, or by recombinantly engineering the nucleic acid encoding a heavy chain of the antibody. Accordingly, a composition of intact antibodies may comprise antibody populations with all K447 residues removed, antibody populations with no K447 residues removed, and antibody populations having a mixture of antibodies with and without the K447 residue. Suitable native-sequence Fc regions for use in the antibodies described herein include human IgG1, IgG2 (IgG2A, IgG2B), IgG3 and IgG4.

As used herein the term "disulfide bond" includes the covalent bond formed between two sulfur atoms. The amino acid cysteine comprises a thiol group that can form a disulfide bond or bridge with a second thiol group. In most naturally occurring IgG molecules, the CH1 and CK regions are linked by a disulfide bond and the two heavy chains are linked by two disulfide bonds at positions corresponding to 239 and 242 using the Kabat numbering system (position 226 or 229, EU numbering system).

As used herein, the term "chimeric antibody" will be held to mean any antibody wherein the immunoreactive region or site is obtained or derived from a first species and the constant region (which may be intact, partial or modified in accordance with the instant disclosure) is obtained from a second species. In certain embodiments the target binding region or site will be from a non-human source (*e.g.* mouse or primate) and the constant region is human.

"Humanized antibody" is used herein to describe an antibody that comprises heavy and light chain variable region sequences from a non-human species (*e.g.* a mouse) but in which at least a portion of the VH and/or VL sequence has been altered to be more "human-like," i.e., more similar to human germline variable sequences. A "humanized antibody" is an antibody or a variant, derivative, analog, or fragment thereof, which immuno-specifically binds to an antigen of interest and which comprises a framework (FR) region having substantially the amino acid sequence of a human antibody and a complementary determining region (CDR) having substantially the amino acid sequence of a non-human antibody. As used herein, the term "substantially" in the context of a CDR refers to a CDR having an amino acid sequence at least 80%, at least 85%, at least 90%, at least 95%, at least 98%, or at least 99% identical to the amino acid sequence of a non-human antibody CDR. A humanized antibody comprises substantially all of at least one, and typically two, variable domains (Fab, Fab', $F(ab')_2$, Fv) in which all or substantially all of the CDR regions correspond to those of a non-human immunoglobulin (*i.e.*, donor antibody) and all or substantially all of the framework regions are those of a human immunoglobulin consensus sequence. In an embodiment, a humanized antibody also comprises at least a portion of an immunoglobulin constant region (Fc), typically that of a human immunoglobulin. In some embodiments, a humanized antibody contains the light chain as well as at least the variable domain of a heavy chain. The antibody also may include the CH1, hinge, CH2, CH3, and CH4 regions of the heavy chain. In some embodiments, a humanized antibody only contains a humanized light chain. In some embodiments, a humanized antibody only contains a humanized heavy chain. In specific embodiments, a humanized antibody only contains a humanized variable domain of a light chain and/or humanized heavy chain.

The term "epitope" as used herein refers to the specific group of atoms or amino acids on an antigen to which an antibody or antibody moiety binds. Two antibodies or antibody moieties may bind the same epitope within an antigen if they exhibit competitive binding for the antigen.

By "specifically binds" or "has specificity to," it is generally meant that an antibody binds to an epitope via its antigen-binding domain, and that the binding entails some complementarity between the antigen-binding domain and the epitope. According to this definition, an antibody is said to "specifically bind" to an epitope when it binds to that epitope, via its antigen-binding domain more readily than it would bind to a random, unrelated epitope. The term "specificity" is used herein to qualify the relative affinity by which a certain antibody binds to a certain epitope. For example, antibody "A" may be deemed to have a higher specificity for a given epitope than antibody "B," or antibody "A" may be said to bind to epitope "C" with a higher specificity than it has for related epitope "D."

As used herein, the terms "treat" or "treatment" refer to both therapeutic treatment and prophylactic or preventative measures, wherein the object is to prevent or slow down (lessen) an undesired physiological change or disorder, such as the progression of cancer. Beneficial or desired clinical results include, but are not limited to, alleviation of symptoms, diminishment of extent of disease, stabilized (*i.e.*, not worsening) state of disease, delay or slowing of disease progression, amelioration or palliation of the disease state, and remission (whether partial or total), whether detectable or undetectable. "Treatment" can also mean prolonging survival as compared to expected survival if not receiving treatment. Those in need of treatment include those already with the condition or disorder as well as those prone to have the condition or disorder or those in which the condition or disorder is to be prevented.

By "subject" or "individual" or "animal" or "patient" or "mammal," is meant any subject, particularly a mammalian subject, for whom diagnosis, prognosis, or therapy is desired. Mammalian subjects include humans, domestic animals, farm animals, and zoo, sport, or pet animals such as dogs, cats, guinea pigs, rabbits, rats, mice, horses, cattle, cows, and so on.

As used herein, phrases such as "to a patient in need of treatment" or "a subject in need of treatment" includes subjects, such as mammalian subjects, that would benefit from administration of an antibody or composition of the present disclosure used, *e.g.*, for detection, for a diagnostic procedure and/or for treatment.

***Anti-DLL3 Antibodies***

As demonstrated in the appended experimental examples, the instant inventors were able to generate anti-DLL3 antibodies 9E8D8, 36B7F3, 129H2B9, 148C3A7, 310P3C5 and 362H3D3 (**Table 1**) all of which have high binding affinity to the human DLL3 protein. The binding is specific as they did not bind to DLL1 or DLL4.

Domain mapping showed that some of these antibodies bound to certain DLL3 domains (**Table A**) not yet targeted before. For instance, 129H2B9 and 148C3A7 bound to the EGF3-4 domain (S312-E389 of UniProt NO. Q9NYJ7) of human DLL3 expressed on cells. 9E8D8 and 310P3C5 specifically bound to the EGF6 domain (R429 to E465 of UniProt NO. Q9NYJ7) of human DLL3 expressed on cells. By contrast, the benchmark antibody DLL3#3 binds the membrane proximal extracellular domain (F466 to L492 of UniProt NO. Q9NYJ7), and the other benchmark antibody DLL3-4-001 specifically binds to EGF3.

***Table A. DLL3 Domain Sequences***

| Domain | Positions | Sequences | SEQ ID NO: |
|---|---|---|---|
| DSL | 176-215 | ARCEPPAVGTACTRLCRPRSAPSRCGPGLRPCAPLEDECE | 118 |
| EGF-like 1 | 216-249 | APLVCRAGCSPEHGFCEQPGECRCLEGWTGPLCT | 119 |
| EGF-like 2 | 274-310 | GPGPCDGNPCANGGSCSETPRSFECTCPRGFYGLRCE | 120 |
| EGF-like 3 | 312-351 | SGVTCADGPCFNGGLCVGGADPDSAYICHCPPGFQGSNCE | 121 |
| EGF-like 4 | 353-389 | RVDRCSLQPCRNGGLCLDLGHALRCRCRAGFAGPRCE | 122 |
| EGF-like 5 | 393-427 | DDCAGRACANGGTCVEGGGAHRCSCALGFGGRDCR | 123 |
| EGF-like 6 | 429-465 | RADPCAARPCAHGGRCYAHFSGLVCACAPGYMGARCE | 124 |
| MPER | 466-492 | FPVHPDGASALPAAPPGLRPGDPQRYL | 125 |

In accordance with one embodiment of the present disclosure, provided is an antibody or antigen-binding fragment thereof. In some embodiments, the antibody or antigen-binding fragment thereof has binding specificity to the human DLL3 protein. In some embodiments, the antibody or antigen-binding fragment thereof includes a heavy chain variable region (VH) that includes a VH CDR1, a VH CDR2 and a VH CDR3, and a light chain variable region (VL) that includes a VL CDR1, a VL CDR2, and a VL CDR3.

In some embodiments, provided is an antibody or antigen-binding fragment that is derived from antibody 310P3C5. In some embodiments, the VH CDR1 includes the amino acid sequence of SEQ ID NO: 37; the VH CDR2 includes the amino acid sequence of SEQ ID NO: 38; the VH CDR3 includes an amino acid sequence selected from the group consisting of SEQ ID NO: 39; the VL CDR1 includes the amino acid sequence of SEQ ID NO: 40; the VL CDR2 includes the amino acid sequence of SEQ ID NO: 41; and the VL CDR3 includes an amino acid sequence selected from the group consisting SEQ ID NO: 42.

In some embodiments, the VH CDR2 is PTM de-risked. The PTM de-risked versions tested include SEQ ID NO:111-117 (**Table 10B**), in which the N residue is substituted with A, F, H, R, V, W or Y In some embodiments, the VH CDR1 includes the amino acid sequence of SEQ ID NO: 37; the VH CDR2 includes the amino acid sequence of SEQ ID NO: 111, 112, 113, 114, 115, 116, or 117; the VH CDR3 includes an amino acid sequence selected from the group consisting of SEQ ID NO: 39; the VL CDR1 includes the amino acid sequence of SEQ ID NO: 40; the VL CDR2 includes the amino acid sequence of SEQ ID NO: 41; and the VL CDR3 includes an amino acid sequence selected from the group consisting SEQ ID NO: 42.

In some embodiments, the VH includes an amino acid sequence selected from the group consisting of SEQ ID NO: 65-69 and 104-110 or a sequence having at least 75%, 80%, 85%, 90%, 95% or 99% sequence identity to any one of SEQ ID NO: 65-69 and 104-110, while retaining the VH CDRs or PTM re-risked versions thereof. In some embodiments, the VL includes an amino acid sequence selected from the group consisting of SEQ ID NO: 70-73 or a sequence having at least 75%, 80%, 85%, 90%, 95% or 99% sequence identity to any one of SEQ ID NO: 70-73 , while retaining the VL CDRs or PTM re-risked versions thereof.

In some embodiments, the VH includes an amino acid sequence selected from the group consisting of SEQ ID NO: 104-110, and the VL includes the amino acid sequence of SEQ ID NO: 73.

Also provided, in some embodiments, are antibodies and antigen-binding fragments therefore that bind to the same epitope on DLL3 as 310P3C5. In some embodiments, provided are antibodies and antigen-binding fragments therefore that bind to the EGF6 domain of the DLL3 protein. Also provided, in some embodiments, are antibodies and antigen-binding fragments therefore that compete with 310P3C5 in binding to DLL3.

In some embodiments, provided is an antibody or antigen-binding fragment that is derived from antibody 9E8D8. In some embodiments, the VH CDR1, VH CDR2, VH CDR3, VL CDR1, VL CDR2 and VL CDR3 include, respectively, the amino acid sequences of SEQ ID NO: 13-18.

In some embodiments, the VH includes the amino acid sequence of SEQ ID NO: 1 or a sequence having at least 75%, 80%, 85%, 90%, 95% or 99% sequence identity to SEQ ID NO: 1, while retaining the VH CDRs or PTM re-risked versions thereof. In some embodiments, the VL includes the amino acid sequence of SEQ ID NO: 2 or a sequence having at least 75%, 80%, 85%, 90%, 95% or 99% sequence identity to SEQ ID NO: 2, while retaining the VL CDRs or PTM re-risked versions thereof.

In some embodiments, the VH includes the amino acid sequence of SEQ ID NO: 1, and the VL includes the amino acid sequence of SEQ ID NO: 2. In some embodiments, the heavy chain includes the amino acid sequence of SEQ ID NO: 49, and the light chain includes the amino acid sequence of SEQ ID NO: 50.

Also provided, in some embodiments, are antibodies and antigen-binding fragments therefore that bind to the same epitope on DLL3 as 9E8D8. In some embodiments, provided are antibodies and antigen-binding fragments therefore that bind to the EGF6 domain of the DLL3 protein. Also provided, in some embodiments, are antibodies and antigen-binding fragments therefore that compete with 9E8D8 in binding to DLL3.

In some embodiments, provided is an antibody or antigen-binding fragment that is derived from antibody 36B7F3. In some embodiments, the VH CDR1, VH CDR2, VH CDR3, VL CDR1, VL CDR2 and VL CDR3 include, respectively, the amino acid sequences of SEQ ID NO: 19-24.

In some embodiments, the VH includes the amino acid sequence of SEQ ID NO: 3 or a sequence having at least 75%, 80%, 85%, 90%, 95% or 99% sequence identity to SEQ ID NO: 3, while retaining the VH CDRs or PTM re-risked versions thereof. In some embodiments, the VL includes the amino acid sequence of SEQ ID NO: 4 or a sequence having at least 75%, 80%, 85%, 90%, 95% or 99% sequence identity to SEQ ID NO: 4, while retaining the VL CDRs or PTM re-risked versions thereof.

In some embodiments, the VH includes the amino acid sequence of SEQ ID NO: 3, and the VL includes the amino acid sequence of SEQ ID NO: 4. In some embodiments, the heavy chain includes the amino acid sequence of SEQ ID NO: 51, and the light chain includes the amino acid sequence of SEQ ID NO: 52.

Also provided, in some embodiments, are antibodies and antigen-binding fragments therefore that bind to the same epitope on DLL3 as 36B7F3. In some embodiments, provided are antibodies and antigen-binding fragments therefore that bind to the membrane proximal extracellular domain of the DLL3 protein. Also provided, in some embodiments, are antibodies and antigen-binding fragments therefore that compete with 36B7F3 in binding to DLL3.

In some embodiments, provided is an antibody or antigen-binding fragment that is derived from antibody 129H2B9. In some embodiments, the VH CDR1, VH CDR2, VH CDR3, VL CDR1, VL CDR2 and VL CDR3 include, respectively, the amino acid sequences of SEQ ID NO: 25-30.

In some embodiments, the VH includes the amino acid sequence of SEQ ID NO: 5 or a sequence having at least 75%, 80%, 85%, 90%, 95% or 99% sequence identity to SEQ ID NO: 5, while retaining the VH CDRs or PTM re-risked versions thereof. In some embodiments, the VL includes the amino acid sequence of SEQ ID NO: 6 or a sequence having at least 75%, 80%, 85%, 90%, 95% or 99% sequence identity to SEQ ID NO: 6, while retaining the VL CDRs or PTM re-risked versions thereof.

In some embodiments, the VH includes the amino acid sequence of SEQ ID NO: 5, and the VL includes the amino acid sequence of SEQ ID NO: 6. In some embodiments, the heavy chain includes the amino acid sequence of SEQ ID NO: 53, and the light chain includes the amino acid sequence of SEQ ID NO: 54.

Also provided, in some embodiments, are antibodies and antigen-binding fragments therefore that bind to the same epitope on DLL3 as 129H2B9. In some embodiments, provided are antibodies and antigen-binding fragments therefore that bind to the EGF3-4 domain of the DLL3 protein. Also provided, in some embodiments, are antibodies and antigen-binding fragments therefore that compete with 129H2B9 in binding to DLL3.

In some embodiments, provided is an antibody or antigen-binding fragment that is derived from antibody 148C3A7. In some embodiments, the VH CDR1, VH CDR2, VH CDR3, VL CDR1, VL CDR2 and VL CDR3 include, respectively, the amino acid sequences of SEQ ID NO: 31-36.

In some embodiments, the VH includes the amino acid sequence of SEQ ID NO: 7 or a sequence having at least 75%, 80%, 85%, 90%, 95% or 99% sequence identity to SEQ ID NO: 7, while retaining the VH CDRs or PTM re-risked versions thereof. In some embodiments, the VL includes the amino acid sequence of SEQ ID NO: 8 or a sequence having at least 75%, 80%, 85%, 90%, 95% or 99% sequence identity to SEQ ID NO: 8, while retaining the VL CDRs or PTM re-risked versions thereof.

In some embodiments, the VH includes the amino acid sequence of SEQ ID NO: 7, and the VL includes the amino acid sequence of SEQ ID NO: 8. In some embodiments, the heavy chain includes the amino acid sequence of SEQ ID NO: 55, and the light chain includes the amino acid sequence of SEQ ID NO: 56.

Also provided, in some embodiments, are antibodies and antigen-binding fragments therefore that bind to the same epitope on DLL3 as 148C3A7. In some embodiments, provided are antibodies and antigen-binding fragments therefore that bind to the EGF3-4 domain of the DLL3 protein. Also provided, in some embodiments, are antibodies and antigen-binding fragments therefore that compete with 148C3A7 in binding to DLL3.

In some embodiments, provided is an antibody or antigen-binding fragment that is derived from antibody 362H3D3. In some embodiments, the VH CDR1, VH CDR2, VH CDR3, VL CDR1, VL CDR2 and VL CDR3 include, respectively, the amino acid sequences of SEQ ID NO: 43-48.

In some embodiments, the VH includes the amino acid sequence of SEQ ID NO: 11 or a sequence having at least 75%, 80%, 85%, 90%, 95% or 99% sequence identity to SEQ ID NO: 11, while retaining the VH CDRs or PTM re-risked versions thereof. In some embodiments, the VL includes the amino acid sequence of SEQ ID NO: 12 or a sequence having at least 75%, 80%, 85%, 90%, 95% or 99% sequence identity to SEQ ID NO: 12, while retaining the VL CDRs or PTM re-risked versions thereof.

In some embodiments, the VH includes the amino acid sequence of SEQ ID NO: 11, and the VL includes the amino acid sequence of SEQ ID NO: 12. In some embodiments, the heavy chain includes the amino acid sequence of SEQ ID NO: 59, and the light chain includes the amino acid sequence of SEQ ID NO: 60.

Also provided, in some embodiments, are antibodies and antigen-binding fragments therefore that bind to the same epitope on DLL3 as 362H3D3. In some embodiments, provided are antibodies and antigen-binding fragments therefore that bind to the membrane proximal extracellular domain of the DLL3 protein. Also provided, in some embodiments, are antibodies and antigen-binding fragments therefore that compete with 362H3D3 in binding to DLL3.

Also provided, in some embodiments, are antibodies and antigen-binding fragments that include CDR sequences derived from the presently disclosed CDR sequences, with one, two or three amino acid substitutions, deletions, and/or additions.

### *Multi-functional Molecules*

Multi-functional molecules that include an antibody or antigen-binding fragment specific to DLL3, such as those disclosed herein, and one or more antibody or antigen-binding fragment having specificity to a second antigen, or a different epitope on DLL3.

In some embodiments, the second antigen is a protein expressed on an immune cell, such as a T cell, a B cell, a monocyte, a macrophage, a neutrophil, a dendritic cell, a phagocyte, a natural killer cell, an eosinophil, a basophil, and a mast cell.

In some embodiments, the second antigen is CD3, CD47, PD1, PD-L1, LAG3, TIM3, CTLA4, VISTA, CSFR1, A2AR, CD73, CD39, CD40, CEA, HER2, CMET, 4-1BB, OX40, SIRPA, CD28, ICOS, CTLA4, BTLA, TIGIT, HVEM, CD27, VEGFR, or VEGF.

Different formats of bispecific antibodies are also provided. In some embodiments, each of the anti-DLL3

fragment and the second fragment each is independently selected from a Fab fragment, a single-chain variable fragment (scFv), or a single-domain antibody. In some embodiments, the bispecific antibody further includes a Fc fragment.

Bifunctional molecules that include not just antibody or antigen binding fragment are also provided. As a tumor antigen targeting molecule, an antibody or antigen-binding fragment specific to DLL3, such as those described here, can be combined with an immune cytokine or ligand optionally through a peptide linker. The linked immune cytokines or ligands include, but not limited to, IL-2, IL-3, IL-4, IL-5, IL-6, IL-7, IL-10, IL-12, IL-13, IL-15, GM-CSF, TNF-$\alpha$, CD40L, OX40L, CD27L, CD30L, 4-1BBL, LIGHT and GITRL. Such bi-functional molecules can combine the immune checkpoint blocking effect with tumor site local immune modulation.

***Chimeric Antigen Receptors***

Also provided, in one embodiment, is a chimeric antigen receptor (CAR) that includes the antibody or fragment thereof of the present disclosure as a targeting unit. In some embodiments, the CAR includes an antibody or fragment thereof of the present disclosure, a transmembrane domain, a costimulatory domain, and a CD3$\varepsilon$ intracellular domain.

A transmembrane domain can be designed to be fused to the extracellular domain which includes the antibody or fragment, optionally through a hinge domain. It can similarly be fused to an intracellular domain, such as a costimulatory domain. In some embodiments, the transmembrane domain can include the natural transmembrane region of a costimulatory domain (*e.g.*, the TM region of a CD28 or 4-1BB employed as a costimulatory domain) or the natural transmembrane domain of a hinge region (*e.g.*, the TM region of a CD8 alpha or CD28 employed as a hinge domain).

In some embodiments, the transmembrane domain can include a sequence that spans a cell membrane, but extends into the cytoplasm of a cell and/or into the extracellular space. For example, a transmembrane can include a membrane-spanning sequence which itself can further include 1, 2, 3, 4, 5, 6, 7, 8, 9, 10 or more amino acids that extend into the cytoplasm of a cell, and/or the extracellular space. Thus, a transmembrane domain includes a membrane-spanning region, yet can further comprise an amino acid(s) that extend beyond the internal or external surface of the membrane itself; such sequences can still be considered to be a "transmembrane domain".

In some embodiments, the transmembrane domain is fused to the cytoplasmic domain through a short linker. Optionally, the short peptide or polypeptide linker, preferably between 2 and 10 amino acids in length can form the linkage between the transmembrane domain and a proximal cytoplasmic signaling domain of the chimeric receptor. A glycine-serine doublet (GS), glycine-serine-glycine triplet (GSG), or alanine- alanine-alanine triplet (AAA) provides a suitable linker.

In some embodiments, the CAR further includes a costimulatory domain. In some embodiments, the costimulatory domain is positioned between the transmembrane domain and an activating domain. Example costimulatory domains include, but are not limited to, CD2, CD3 delta, CD3 epsilon, CD3 gamma, CD4, CD7, CD8a, CD8 , CD11a (ITGAL), CD11b (ITGAM), CD11c (ITGAX), CD11d (ITGAD), CD18 (ITGB2), CD19 (B4), CD27 (T FRSF7), CD28, CD28T, CD29 (ITGB1), CD30 (TNFRSF8), CD40 (TNFRSF5), CD48 (SLAMF2), CD49a (ITGA1), CD49d (ITGA4), CD49f (ITGA6), CD66a (CEACAM1), CD66b (CEACAM8), CD66c (CEACAM6), CD66d (CEACAM3), CD66e (CEACAM5), CD69 (CLEC2), CD79A (B-cell antigen receptor complex-associated alpha chain), CD79B (B-cell antigen receptor complex- associated beta chain), CD84 (SLAMFS), CD96 (Tactile), CD 100 (SEMA4D), CD 103 (ITGAE), CD134 (OX40), CD137 (4-1BB), CD150 (SLAMF1), CD158A (KIR2DL1), CD158B1 (KIR2DL2), CD158B2 (KIR2DL3), CD158C (KIR3DP1), CD158D (KIRDL4), CD158F1 (KIR2DL5A), CD158F2 (KIR2DL5B), CD158K (KTR3DL2), CD160 (BY55), CD162 (SELPLG), CD226 (DNAM1), CD229 (SLAMF3), CD244 (SLAMF4), CD247 (CD3-zeta), CD258 (LIGHT), CD268 (BAFFR), CD270 (T FSF14), CD272 (BTLA), CD276 (B7-H3), CD279 (PD-1), CD314 (KG2D), CD319 (SLAMF7), CD335 (K-p46), CD336 ( K-p44), CD337 ( K-p30), CD352 (SLAMF6), CD353 (SLAMF8), CD355 (CRTAM), CD357 (TNFRSF 18), inducible T cell co-stimulator (ICOS), LFA-1 (CD11a/CD18), KG2C, DAP-10, ICAM-1, Kp80 (KLRF1), IL-2R beta, IL-2R gamma, IL-7R alpha, LFA-1, SLAMF9, LAT, GADS (GrpL), SLP-76 (LCP2), PAG1/CBP, a CD83 ligand, Fc gamma receptor, MHC class 1 molecule, MHC class 2 molecule, a TNF receptor protein, an immunoglobulin protein, a cytokine receptor, an integrin, activating NK cell receptors, a Toll ligand receptor, and fragments or combinations thereof.

In some embodiments, the cytoplasmic portion of the CAR also includes a signaling/activation domain. In one embodiment, the signaling/activation domain is the CD3$\varepsilon$ domain, or is an amino acid sequence having at least about 80%, 85%, 90%, 95%, 98% or 99% sequence identity to the CD3$\varepsilon$ domain.

***Polynucleotides, mRNA, and Methods of Expressing or Preparing Antibodies***

The present disclosure also provides polynucleotides or nucleic acid molecules encoding the antibodies, variants or derivatives thereof of the disclosure, or the CAR. The polynucleotides of the present disclosure may encode the entire heavy and light chain variable regions of the antigen-binding polypeptides, variants or derivatives thereof on the same polynucleotide molecule or on separate polynucleotide molecules. Additionally, the polynucleotides of the present disclosure may encode portions of the heavy and light chain variable regions of the antigen-binding polypeptides, variants

or derivatives thereof on the same polynucleotide molecule or on separate polynucleotide molecules.

In some embodiments, the polynucleotide is an mRNA molecule. In some embodiments, the mRNA can be introduced into a target cell for expressing the antibody or fragment thereof.

mRNAs may be synthesized according to any of a variety of known methods. For example, the mRNAs may be synthesized via *in vitro* transcription (IVT). Briefly, IVT is typically performed with a linear or circular DNA template containing a promoter, a pool of ribonucleotide triphosphates, a buffer system that may include DTT and magnesium ions, and an appropriate RNA polymerase (e.g., T3, T7 or SP6 RNA polymerase), DNAse I, pyrophosphatase, and/or RNAse inhibitor. The exact conditions will vary according to the specific application.

In some embodiments, for the preparation of antibody-coding mRNA, a DNA template is transcribed *in vitro.* A suitable DNA template typically has a promoter, for example a T3, T7 or SP6 promoter, for in vitro transcription, followed by desired nucleotide sequence for desired antibody encoding (*e.g.*, heavy chain or light chain encoding) mRNA and a termination signal.

Desired antibody encoding (*e.g.*, heavy chain or light chain encoding) mRNA sequence may be determined and incorporated into a DNA template using standard methods. For example, starting from a desired amino acid sequence (*e.g.*, a desired heavy chain or light chain sequence), a virtual reverse translation is carried out based on the degenerated genetic code. Optimization algorithms may then be used for selection of suitable codons. Typically, the G/C content can be optimized to achieve the highest possible G/C content on one hand, taking into the best possible account the frequency of the tRNAs according to codon usage on the other hand. The optimized RNA sequence can be established and displayed, for example, with the aid of an appropriate display device and compared with the original (wild-type) sequence. A secondary structure can also be analyzed to calculate stabilizing and destabilizing properties or, respectively, regions of the RNA.

The mRNA may be synthesized as unmodified or modified mRNA. Typically, mRNAs are modified to enhance stability. Modifications of mRNA can include, for example, modifications of the nucleotides of the RNA. A modified mRNA can thus include, for example, backbone modifications, sugar modifications or base modifications. In some embodiments, antibody encoding mRNAs (*e.g.*, heavy chain and light chain encoding mRNAs) may be synthesized from naturally occurring nucleotides and/or nucleotide analogues (modified nucleotides) including, but not limited to, purines (adenine (A), guanine (G)) or pyrimidines (thymine (T), cytosine (C), uracil (U)), and as modified nucleotides analogues or derivatives of purines and pyrimidines, such as *e.g.* 1-methyl-adenine, 2-methyl-adenine, 2-methylthio-N-6-isopentenyl-adenine, N6-methyl-adenine, N6-isopentenyl-adenine, 2-thio-cytosine, 3-methyl-cytosine, 4-acetyl-cytosine, 5-methyl-cytosine, 2,6-diaminopurine, 1-methyl-guanine, 2-methyl-guanine, 2,2-dimethyl-guanine, 7-methyl-guanine, inosine, 1-methyl-inosine, pseudouracil (5-uracil), dihydro-uracil, 2-thio-uracil, 4-thio-uracil, 5-carboxymethylaminomethyl-2-thio-uracil, 5-(carboxyhydroxymethyl)-uracil, 5-fluoro-uracil, 5-bromo-uracil, 5-carboxymethylaminomethyl-uracil, 5-methyl-2-thio-uracil, 5-methyl-uracil, N-uracil-5-oxyacetic acid methyl ester, 5-methylaminomethyl-uracil, 5-methoxyaminomethyl-2-thio-uracil, 5'-methoxycarbonylmethyl-uracil, 5-methoxy-uracil, uracil-5-oxyacetic acid methyl ester, uracil-5-oxyacetic acid (v), 1-methyl-pseudouracil, queosine, 13-D-mannosyl-queosine, wybutoxosine, and phosphoramidates, phosphorothioates, peptide nucleotides, methylphosphonates, 7-deazaguanosine, 5-methylcytosine and inosine. The preparation of such analogues is known to a person skilled in the art e.g. from the U.S. Pat. Nos. 4,373,071, 4,401,796, 4,415,732, 4,458,066, 4,500,707, 4,668,777, 4,973,679, 5,047,524, 5,132,418, 5,153,319, 5,262,530 and 5,700,642, the disclosure of which is included here in its full scope by reference.

In some embodiments, the mRNAs (*e*.g., heavy chain and light chain encoding mRNAs) may contain RNA backbone modifications. Typically, a backbone modification is a modification in which the phosphates of the backbone of the nucleotides contained in the RNA are modified chemically. Exemplary backbone modifications typically include, but are not limited to, modifications from the group consisting of methylphosphonates, methylphosphoramidates, phosphoramidates, phosphorothioates (*e.g.* cytidine 5'-O-(1-thiophosphate)), boranophosphates, positively charged guanidinium groups etc., which means by replacing the phosphodiester linkage by other anionic, cationic or neutral groups.

In some embodiments, the mRNAs (*e.g.*, heavy chain and light chain encoding mRNAs) may contain sugar modifications. A typical sugar modification is a chemical modification of the sugar of the nucleotides it contains including, but not limited to, sugar modifications chosen from the group consisting of 2'-deoxy-2'-fluoro-oligoribonucleotide (2'-fluoro-2'-deoxycytidine 5'-triphosphate, 2'-fluoro-2'-deoxyuridine 5'-triphosphate), 2'-deoxy-2'-deamine-oligoribonucleotide (2'-amino-2'-deoxycytidine 5'-triphosphate, 2'-amino-2'-deoxyuridine 5'-triphosphate), 2'-O-alkyloligoribonucleotide, 2'-deoxy-2'-C-alkyloligoribonucleotide (2'-O-methylcytidine 5'-triphosphate, 2'-methyluridine 5'-triphosphate), 2'-C-alkyloligoribonucleotide, and isomers thereof (2'-aracytidine 5'-triphosphate, 2'-arauridine 5'-triphosphate), or azidotriphosphates (2'-azido-2'-deoxycytidine 5'-triphosphate, 2'-azido-2'-deoxyuridine 5'-triphosphate).

In some embodiments, the mRNAs (*e.g.*, heavy chain and light chain encoding mRNAs) may contain modifications of the bases of the nucleotides (base modifications). A modified nucleotide which contains a base modification is also called a base-modified nucleotide. Examples of such base-modified nucleotides include, but are not limited to, 2-amino-6-chloropurine riboside 5'-triphosphate, 2-aminoadenosine 5'-triphosphate, 2-thiocytidine 5'-triphosphate, 2-thiouridine 5'-triphosphate, 4-thiouridine 5'-triphosphate, 5-aminoallylcytidine 5'-triphosphate, 5-aminoallyluridine 5'-triphosphate, 5-

bromocytidine 5'-triphosphate, 5-bromouridine 5'-triphosphate, 5-iodocytidine 5'-triphosphate, 5-iodouridine 5'-triphosphate, 5-methylcytidine 5'-triphosphate, 5-methyluridine 5'-triphosphate, 6-azacytidine 5'-triphosphate, 6-azauridine 5'-triphosphate, 6-chloropurine riboside 5'-triphosphate, 7-deazaadenosine 5'-triphosphate, 7-deazaguanosine 5'-triphosphate, 8-azaadenosine 5'-triphosphate, 8-azidoadenosine 5'-triphosphate, benzimidazole riboside 5'-triphosphate, N1-methyladenosine 5'-triphosphate, N1-methylguanosine 5'-triphosphate, N6-methyladenosine 5'-triphosphate, O6-methylguanosine 5'-triphosphate, pseudouridine 5'-triphosphate, puromycin 5'-triphosphate or xanthosine 5'-triphosphate.

Typically, mRNA synthesis includes the addition of a "cap" on the N-terminal (5') end, and a "tail" on the C-terminal (3') end. The presence of the cap is important in providing resistance to nucleases found in most eukaryotic cells. The presence of a "tail" serves to protect the mRNA from exonuclease degradation.

Thus, in some embodiments, the mRNAs (*e.g.*, heavy chain and light chain encoding mRNAs) include a 5' cap structure. A 5' cap is typically added as follows: first, an RNA terminal phosphatase removes one of the terminal phosphate groups from the 5' nucleotide, leaving two terminal phosphates; guanosine triphosphate (GTP) is then added to the terminal phosphates via a guanylyl transferase, producing a 5'5'5 triphosphate linkage; and the 7-nitrogen of guanine is then methylated by a methyltransferase. Examples of cap structures include, but are not limited to, m7G(5')ppp (5'(A,G(5')ppp(5)A and G(5)ppp(5')G.

In some embodiments, the mRNAs (*e.g.*, heavy chain and light chain encoding mRNAs) include a 3' poly(A) tail structure. A poly-A tail on the 3' terminus of mRNA typically includes about 10 to 300 adenosine nucleotides (*e.g.*, about 10 to 200 adenosine nucleotides, about 10 to 175 adenosine nucleotides, about 10 to 150 adenosine nucleotides, about 10 to 125 adenosine nucleotides, 10 to 100 adenosine nucleotides, about 10 to 75 adenosine nucleotides, about 20 to 70 adenosine nucleotides, or about 20 to 60 adenosine nucleotides). In some embodiments, antibody encoding mRNAs (*e.g.*, heavy chain and light chain encoding mRNAs) include a 3' poly(C) tail structure. A suitable poly-C tail on the 3' terminus of mRNA typically include about 10 to 200 cytosine nucleotides (*e.g.*, about 10 to 150 cytosine nucleotides, about 10 to 100 cytosine nucleotides, about 20 to 70 cytosine nucleotides, about 20 to 60 cytosine nucleotides, or about 10 to 40 cytosine nucleotides). The poly-C tail may be added to the poly-A tail or may substitute the poly-A tail.

In some embodiments, the mRNAs (*e.g.*, heavy chain and light chain encoding mRNAs) include a 5' and/or 3' untranslated region. In some embodiments, a 5' untranslated region includes one or more elements that affect an mRNA's stability or translation, for example, an iron responsive element. In some embodiments, a 5' untranslated region may be between about 50 and 500 nucleotides in length (*e.g.*, about 50 and 400 nucleotides in length, about 50 and 300 nucleotides in length, about 50 and 200 nucleotides in length, or about 50 and 100 nucleotides in length).

In some embodiments, a 5' region of an mRNA (*e.g.*, heavy chain and light chain encoding mRNAs) includes a sequence encoding a signal peptide, such as those described herein. In particular embodiments, a signal peptide derived from human growth hormone (hGH) is incorporated in the 5' region. Typically, a signal peptide encoding sequence is linked, directly or indirectly, to the heavy chain or light chain encoding sequence at the N-terminus.

The present technology may be used to deliver any antibody known in the art and antibodies that can be produced against desired antigens using standard methods. The present invention may be used to deliver monoclonal antibodies, polyclonal antibodies, antibody mixtures or cocktails, human or humanized antibodies, chimeric antibodies, or bi-specific antibodies.

Methods of making antibodies are well known in the art and described herein. In certain embodiments, both the variable and constant regions of the antigen-binding polypeptides of the present disclosure are fully human. Fully human antibodies can be made using techniques described in the art and as described herein. For example, fully human antibodies against a specific antigen can be prepared by administering the antigen to a transgenic animal which has been modified to produce such antibodies in response to antigenic challenge, but whose endogenous loci have been disabled. Exemplary techniques that can be used to make such antibodies are described in U.S. patents: 6,150,584; 6,458,592; 6,420,140 which are incorporated by reference in their entireties.

***Treatment and*** *Uses*

As described herein, the antibodies, variants, derivatives or antibody-drug conjugates of the present disclosure may be used in certain treatment and diagnostic methods.

The present disclosure is further directed to antibody-based therapies which involve administering the antibodies, fragments, or antibody-drug conjugates of the disclosure to a patient such as an animal, a mammal, and a human for treating one or more of the disorders or conditions described herein. Therapeutic compounds of the disclosure include, but are not limited to, antibodies of the disclosure (including variants and derivatives thereof as described herein) and nucleic acids or polynucleotides encoding antibodies of the disclosure (including variants and derivatives thereof as described herein).

The antibodies of the disclosure can also be used to treat or inhibit cancer. As provided above, DLL3 can be overexpressed in tumor cells, in particular liver, gastric, pancreatic, esophageal, ovarian, and lung tumors. Inhibition of

DLL3 has been shown to be useful for treating the tumors.

Accordingly, in some embodiments, provided are methods for treating a cancer in a patient in need thereof. The method, in one embodiment, entails administering to the patient an effective amount of an antibody, fragment, or antibody-drug conjugate of the present disclosure. In some embodiments, at least one of the cancer cells (*e.g.*, stromal cells) in the patient over-express DLL3.

Cellular therapies, such as chimeric antigen receptor (CAR) T-cell therapies, are also provided in the present disclosure. A suitable cell can be used, that is transduced with a vector that encodes, or put in contact with, an CAR that includes an anti-DLL3 antibody of the present disclosure (or alternatively engineered to express an anti-DLL3 antibody of the present disclosure). Upon such contact or engineering, the cell can then be introduced to a cancer patient in need of a treatment. The cancer patient may have a cancer of any of the types as disclosed herein. The cell (*e.g.*, T cell) can be, for instance, a tumor-infiltrating T lymphocyte, a CD4+ T cell, a CD8+ T cell, or the combination thereof, without limitation.

In some embodiments, the cell was isolated from the cancer patient him- or her-self. In some embodiments, the cell was provided by a donor or from a cell bank. When the cell is isolated from the cancer patient, undesired immune reactions can be minimized.

Non-limiting examples of cancers include bladder cancer, breast cancer, colorectal cancer, endometrial cancer, esophageal cancer, head and neck cancer, kidney cancer, leukemia, liver cancer, lung cancer, lymphoma, melanoma, pancreatic cancer, prostate cancer, and thyroid cancer. In some embodiments, the cancer is one or more of gastric, pancreatic, esophageal, ovarian, lung cancers and cutaneous T cell lymphoma. In some embodiments, the cancer is small cell lung cancer (SCLC).

Additional diseases or conditions associated with increased cell survival, that may be treated, prevented, diagnosed and/or prognosed with the antibodies or variants, or derivatives thereof of the disclosure include, but are not limited to, progression, and/or metastases of malignancies and related disorders such as leukemia (including acute leukemias (*e.g.*, acute lymphocytic leukemia, acute myelocytic leukemia (including myeloblastic, promyelocytic, myelomonocytic, monocytic, and erythroleukemia)) and chronic leukemias (*e.g.*, chronic myelocytic (granulocytic) leukemia and chronic lymphocytic leukemia)), polycythemia vera, lymphomas (*e.g.*, Hodgkin's disease and non-Hodgkin's disease), multiple myeloma, Waldenstrom's macroglobulinemia, heavy chain disease, and solid tumors including, but not limited to, sarcomas and carcinomas such as fibrosarcoma, myxosarcoma, liposarcoma, chondrosarcoma, osteogenic sarcoma, chordoma, angiosarcoma, endotheliosarcoma, lymphangiosarcoma, lymphangioendotheliosarcoma, synovioma, mesothelioma, Ewing's tumor, leiomyosarcoma, rhabdomyo sarcoma, colon carcinoma, pancreatic cancer, breast cancer, ovarian cancer, prostate cancer, squamous cell carcinoma, basal cell carcinoma, adenocarcinoma, sweat gland carcinoma, sebaceous gland carcinoma, papillary carcinoma, papillary adenocarcinomas, cystadenocarcinoma, medullary carcinoma, bronchogenic carcinoma, renal cell carcinoma, hepatoma, bile duct carcinoma, choriocarcinoma, seminoma, embryonal carcinoma, Wilm's tumor, cervical cancer, testicular tumor, lung carcinoma, small cell lung carcinoma, bladder carcinoma, epithelial carcinoma, glioma, astrocytoma, medulloblastoma, craniopharyngioma, ependymoma, pinealoma, hemangioblastoma, acoustic neuroma, oligodendroglioma, menangioma, melanoma, neuroblastoma and retinoblastoma.

A specific dosage and treatment regimen for any particular patient will depend upon a variety of factors, including the particular antibodies, variant or derivative thereof used, the patient's age, body weight, general health, sex, and diet, and the time of administration, rate of excretion, drug combination, and the severity of the particular disease being treated. Judgment of such factors by medical caregivers is within the ordinary skill in the art. The amount will also depend on the individual patient to be treated, the route of administration, the type of formulation, the characteristics of the compound used, the severity of the disease, and the desired effect. The amount used can be determined by pharmacological and pharmacokinetic principles well known in the art.

Methods of administration of the antibody, fragment, or antibody-drug conjugate or include but are not limited to intradermal, intramuscular, intraperitoneal, intravenous, subcutaneous, intranasal, epidural, and oral routes. The antigen-binding polypeptides or compositions may be administered by any convenient route, for example by infusion or bolus injection, by absorption through epithelial or mucocutaneous linings (*e.g.*, oral mucosa, rectal and intestinal mucosa, etc.) and may be administered together with other biologically active agents. Thus, pharmaceutical compositions containing the antigen-binding polypeptides of the disclosure may be administered orally, rectally, parenterally, intracisternally, intravaginally, intraperitoneally, topically (as by powders, ointments, drops or transdermal patch), buccally, or as an oral or nasal spray.

The term "parenteral" as used herein refers to modes of administration which include intravenous, intramuscular, intraperitoneal, intrasternal, subcutaneous and intra-articular injection and infusion.

Administration can be systemic or local. In addition, it may be desirable to introduce the antibodies of the disclosure into the central nervous system by any suitable route, including intraventricular and intrathecal injection; intraventricular injection may be facilitated by an intraventricular catheter, for example, attached to a reservoir, such as an Ommaya reservoir. Pulmonary administration can also be employed, *e.g.*, by use of an inhaler or nebulizer, and formulation with an aerosolizing agent.

It may be desirable to administer the antigen-binding polypeptides or compositions of the disclosure locally to the area in need of treatment; this may be achieved by, for example, and not by way of limitation, local infusion during surgery, topical application, *e.g.*, in conjunction, with a wound dressing after surgery, by injection, by means of a catheter, by means of a suppository, or by means of an implant, said implant being of a porous, non-porous, or gelatinous material, including membranes, such as sialastic membranes, or fibers. Preferably, when administering a protein, including an antibody, of the disclosure, care must be taken to use materials to which the protein does not absorb.

The amount of the antibodies, fragments, or antibody-drug conjugates of the disclosure which will be effective in the treatment, inhibition and prevention of an inflammatory, immune or malignant disease, disorder or condition can be determined by standard clinical techniques. In addition, *in vitro* assays may optionally be employed to help identify optimal dosage ranges. The precise dose to be employed in the formulation will also depend on the route of administration, and the seriousness of the disease, disorder or condition, and should be decided according to the judgment of the practitioner and each patient's circumstances. Effective doses may be extrapolated from dose-response curves derived from *in vitro* or animal model test systems.

As a general proposition, the dosage administered to a patient of the antibodies, fragments, or antibody-drug conjugates of the present disclosure is typically 0.001 mg/kg to 100 mg/kg of the patient's body weight, between 0.01 mg/kg and 20 mg/kg of the patient's body weight, or 0.5 mg/kg to 10 mg/kg of the patient's body weight. Generally, human antibodies have a longer half-life within the human body than antibodies from other species due to the immune response to the foreign polypeptides. Thus, lower dosages of human antibodies and less frequent administration is often possible. Further, the dosage and frequency of administration of antibodies of the disclosure may be reduced by enhancing uptake and tissue penetration (*e.g.*, into the brain) of the antibodies by modifications such as, for example, lipidation.

In an additional embodiment, the compositions of the disclosure are administered in combination with cytokines. Cytokines that may be administered with the compositions of the disclosure include, but are not limited to, IL-2, IL-3, IL-4, IL-5, IL-6, IL-7, IL-10, IL-12, IL-13, IL-15, anti-CD40, CD40L, and TNF-$\alpha$.

In additional embodiments, the compositions of the disclosure are administered in combination with other therapeutic or prophylactic regimens, such as, for example, radiation therapy.

***Compositions***

The present disclosure also provides pharmaceutical compositions. Such compositions comprise an effective amount of an antibody, fragment, or antibody-drug conjugate, and an acceptable carrier. In some embodiments, the composition further includes a second anticancer agent (*e.g.*, an immune checkpoint inhibitor).

In a specific embodiment, the term "pharmaceutically acceptable" means approved by a regulatory agency of the Federal or a state government or listed in the U.S. Pharmacopeia or other generally recognized pharmacopeia for use in animals, and more particularly in humans. Further, a "pharmaceutically acceptable carrier" will generally be a non-toxic solid, semisolid or liquid filler, diluent, encapsulating material or formulation auxiliary of any type.

The term "carrier" refers to a diluent, adjuvant, excipient, or vehicle with which the therapeutic is administered. Such pharmaceutical carriers can be sterile liquids, such as water and oils, including those of petroleum, animal, vegetable or synthetic origin, such as peanut oil, soybean oil, mineral oil, sesame oil and the like. Water is a preferred carrier when the pharmaceutical composition is administered intravenously. Saline solutions and aqueous dextrose and glycerol solutions can also be employed as liquid carriers, particularly for injectable solutions. Suitable pharmaceutical excipients include starch, glucose, lactose, sucrose, gelatin, malt, rice, flour, chalk, silica gel, sodium stearate, glycerol monostearate, talc, sodium chloride, dried skim milk, glycerol, propylene, glycol, water, ethanol and the like. The composition, if desired, can also contain minor amounts of wetting or emulsifying agents, or pH buffering agents such as acetates, citrates or phosphates. Antibacterial agents such as benzyl alcohol or methyl parabens; antioxidants such as ascorbic acid or sodium bisulfite; chelating agents such as ethylenediaminetetraacetic acid; and agents for the adjustment of tonicity such as sodium chloride or dextrose are also envisioned. These compositions can take the form of solutions, suspensions, emulsion, tablets, pills, capsules, powders, sustained-release formulations and the like. The composition can be formulated as a suppository, with traditional binders and carriers such as triglycerides. Oral formulation can include standard carriers such as pharmaceutical grades of mannitol, lactose, starch, magnesium stearate, sodium saccharine, cellulose, magnesium carbonate, etc. Examples of suitable pharmaceutical carriers are described in Remington's Pharmaceutical Sciences by E. W. Martin, incorporated herein by reference. Such compositions will contain a therapeutically effective amount of the antigen-binding polypeptide, preferably in purified form, together with a suitable amount of carrier so as to provide the form for proper administration to the patient. The formulation should suit the mode of administration. The parental preparation can be enclosed in ampoules, disposable syringes or multiple dose vials made of glass or plastic.

In an embodiment, the composition is formulated in accordance with routine procedures as a pharmaceutical composition adapted for intravenous administration to human beings. Typically, compositions for intravenous administration are solutions in sterile isotonic aqueous buffer. Where necessary, the composition may also include a solubilizing

agent and a local anesthetic such as lignocaine to ease pain at the site of the injection. Generally, the ingredients are supplied either separately or mixed together in unit dosage form, for example, as a dry lyophilized powder or water free concentrate in a hermetically sealed container such as an ampoule or sachet indicating the quantity of active agent. Where the composition is to be administered by infusion, it can be dispensed with an infusion bottle containing sterile pharmaceutical grade water or saline. Where the composition is administered by injection, an ampoule of sterile water for injection or saline can be provided so that the ingredients may be mixed prior to administration.

## EXAMPLES

### Example 1. Generation of Mouse Anti Human DLL3 Antibody

This example describes the generation of mouse anti-human DLL3 monoclonal antibodies using the hybridoma technology.

*Immunogen:* Two immunogens, one were used during the mouse immunization process. One is comprised of the extracellular domain (ECD, A27-L492 of UniProt NO. Q9NYJ7) of human DLL3 protein fused with a human Fc fragment (hDLL3-hFc, Acro Bio, Cat. No. DL3-H5255). The other is the ECD of human DLL3 protein fused with his tag (hDLL3-his, Acro Bio, Cat. No. DL3-H52H4).

*Scheme of mouse immunization:* To generate mouse monoclonal antibodies to human DLL3, BALB/c and C57BL/6 mice were immunized with the hDLL3-hFc or hDLL3-his protein at a biweekly interval intraperitoneally or subcutaneously. Serum titers of immunized mice were monitored by ELISA against the human hDLL3-his protein. After several rounds of immunization, mice with sufficient titers were boosted with the hDLL3-his protein and selected for fusion.

*Cell fusion and hybridoma screening:* Splenocytes from the selected mice were fused with mouse myeloma cell line Sp2/0 by electrofusion. These hybridoma cells were then plated in 96 flat-bottom microplates and secreted mouse antibodies in the supernatant. During primary screening, proteins binding to hDLL3-his by ELISA and cells binding to the ECD of human DLL3 overexpressed on HEK293 cells (HEK293-hDLL3, customized by Genomeditech) or SCLC cell line SHP77 (ATCC, Cat: NO. CRL-2195) with constitutive DLL3 expression by FACS were used to screen positive clones in a high-throughput manner. A following confirmative screening was pursued to filter out clones with non-specific binding to the human DLL1 his tag fusion protein (hDLL1-his, Sino Biological, Cat. No. 11635-H08H) or the human DLL4 his tag fusion protein (hDLL4-his, Sino Biologial, Cat. No. 10171-H08H) by ELISA. Clones binding to cynomolgus DLL3 his tag fusion protein (cynoDLL3-his, Acro Bio, Cat. No. DL3-C52H3) were identified by ELISA.

*Subcloning screening and sequencing:* Positive primary clones meeting the above criteria from each fusion were subcloned by limiting dilution to ensure that hybridoma subclones were derived from a single parental cell. Subclones were screened with the same criteria as the primary clones as described above. The subclones with specific binding potency to hDLL3 and cynoDLL3 and no binding to hDLL1 and hDLL4 were selected for subsequent sequencing.

The resulting sequences of the variable region of the mouse antibodies were fused with the constant region of human IgG1 to generate chimeric DLL3 mAbs. The DNA sequences of chimeric antibodies were cloned into the pcDNA3.4 plasmid and expressed in CHO-K1 cells followed by purification of antibodies by Protein A affinity chromatography column or beads. The purified chimeric antibodies were subjected to serial *in vitro* screening to determine affinity, binding ability, specificity, and species cross-reactivity.

Six chimeric mAbs, including 9E8D8, 36B7F3, 129H2B9, 148C3A7, 310P3C5 and 362H3D3, were selected for further analysis based on the performance in screening assays. The amino acid sequences of the variable regions of the selected chimeric DLL3 antibodies are provided in **Table 1** as below, with the CDR sequences summarized in **Table 2.** The sequences of the heavy chain and light chain of all the antibodies are listed in **Table 3.** Two benchmark DLL3 mAbs, DLL3-4-001 (CC) (sequence derived from the IMGT database) and DLL3#3 (see WO2019234220), are their respective DLL3 binding elements of two T cell engagers (TCE) Tarlatamab and BI765432 undergoing clinical evaluation.

***Table 1. Variable region Sequences of DLL3 chimeric mAbs (underline indicates CDRs)***

| Name | Sequence | SEQ NO ID: |
|---|---|---|
| 9E8D8 VH | QVQLQQSGAELVRPGASVKLSCKALGYTFT**DSEIH**WVKQTPVHGLEWIG**AIHPRNGGTAYNQKFRG**KATLSADRSSNTAYMELSSLTSEDSAVYYCSR**GYFLDY**WGQGTSVTVSS | 1 |
| 9E8D8 VL | DIVMTQSPSSLTMSVGQKVTMSC**KSSQSLLNSDNQKNYLA**WYQQRPGQSPKLLLY**FASTRES**GVPDRFIGSGSGTDFTLTISSVQAEDLADYFC**QHHYDTPPT**FGGGTKLEIK | 2 |

(continued)

| Name | Sequence | SEQ NO ID: |
|---|---|---|
| 36B7F3 VH | QVQLQQSGPELVKPGASVKISCRASGYTFT**DHHLN**WVKQRPGQGLEWIG**WIFPGSGSDYYNERFKD**KATLTVDKSSSTAYMLLSSLTSEDSAVYFCER**WDY**WGQGTTLTVSS | 3 |
| 36B7F3 VL | QIVLTQSPTIMSASPGEKVTMTC**SASSSISYMH**WYQQKPGTSPKRWIY**DTSTLAS**GVPARFSGGGSGTSYSLTISSMEAEDAATYYC**HQRSSYPLT**FGAGTKLELK | 4 |
| 129H2B9 VH | EVNLEESGGGLVQPGGSMKLSCVVSGFTFS**NYWMS**WVRQSPEKGLEWVA**EIRLKSDNYATHYAESVKG**KFTISRDDSKSRLYLQMNSLRPEDTGIYYCTS**NRFAY**WGQGTLVTVSA | 5 |
| 129H2B9 VL | NIVMTQSPKSVSMSVGERVTLSC**KASENVGTYVY**WYQQKPEQSPKMMIY**GASNRYT**GVPDRFTGSGSATDFTLTIRSVQAEDLADYHC**GQSYSYPFT**FGSGTKLEIK | 6 |
| 148C3A7 VH | EVQLQQSGPELIKPGTSVKMSCKASGYTFT**TYLMH**WVKQKPGQGLEWIG**YINPYNDVTEYHEKFKD**KATLTSDKSSSTAYMELSSLTSEDSAVYYCAR**LGGYGPGWYLDV**WGAGTTVTVSS | 7 |
| 148C3A7 VL | DIQMTQSPASLSASVGETVTITC**RASGSIHNYLA**WYLHKQGKSPQLLVY**NAKTLAD**GVPSRFSGSGSGTQYSLKINSLQPEDFGSYYC**HHFWSTPWT**FGGGTKLEIK | 8 |
| 310P3C5 VH | EVQLQQSGPELVKPGASVKISCKASGYTFT**DFYMN**WVKQTHGKSLEWIG**DANPNNGGTTHNPRFKG**QATLTVDKSSNTAYMELRSLTSEDSAVYYCAR**GGLPGDFDY**WGQGTTLTVSS | 9 |
| 310P3C5 VL | DIQMTQSSSSFSVSLGDRVTITC**KASEDIYNRLA**WYQQKPGNAPRLLIS**GVTSLET**GVPSRFSGSGSGKDYTLSITSLQTEDVATYYC**QQYWSFPWT**FGGGTKLEIK | 10 |
| 362H3D3 VH | EVQLQQSGPELLKPGASVKISCKASGYTFT**DYTMH**WVKQSHGRSLEWIG**GIHPNYGGTSYNEKFKD**KATLTVDKSSSTAYMEFRSLTSEDSAVYYCAR**WGYYGGSYWYFDV**WGTGTTVTVSS | 11 |
| 362H3D3 VL | DIQMTQSPASLSVSVGETVTITC**RASENIYSNLA**WYQQKQGKSPQLLVY**AATNLAD**GVPSRFSGSGSGTQYSLKINSLQSEDFGSYYC**QHFWGTPYT**FGGGTKLEIK | 12 |

*Table 2. CDR Sequences (Kabat numbering)*

| Antibody | VH CDR1 | SEQ NO ID: | VH CDR2 | SEQ NO ID: | VH CDR3 | SEQ NO ID: |
|---|---|---|---|---|---|---|
| 9E8D8 | DSEIH | 13 | AIHPRNGGTAYNQKFRG | 14 | GYFLDY | 15 |
| 36B7F3 | DHHLN | 19 | WIFPGSGSDYYNERFKD | 20 | WDY | 21 |
| 129H2B9 | NYWMS | 25 | EIRLKSDNYATHYAESVKG | 26 | NRFAY | 27 |
| 148C3A7 | TYLMH | 31 | YINPYNDVTEYHEKFKD | 32 | LGGYGPGWYLDV | 33 |
| 310P3C5 | DFYMN | 37 | DANPNNGGTTHNPRFKG | 38 | GGLPGDFDY | 39 |
| 362H3D3 | DYTMH | 43 | GIHPNYGGTSYNEKFKD | 44 | WGYYGGSYWYFDV | 45 |

| Antibody | VL CDR1 | SEQ NO ID: | VL CDR2 | SEQ NO ID: | VL CDR3 | SEQ NO ID: |
|---|---|---|---|---|---|---|
| 9E8D8 | KSSQSLLNSDNQKNYLA | 16 | FASTRES | 17 | QHHYDTPPT | 18 |

(continued)

| Antibody | VL CDR1 | SEQ NO ID: | VL CDR2 | SEQ NO ID: | VL CDR3 | SEQ NO ID: |
|---|---|---|---|---|---|---|
| 36B7F3 | SASSSISYMH | 22 | DTSTLAS | 23 | HQRSSYPLT | 24 |
| 129H2B9 | KASENVGTYVY | 28 | GASNRYT | 29 | GQSYSYPFT | 30 |
| 148C3A7 | RASGSIHNYLA | 34 | NAKTLAD | 35 | HHFWSTPWT | 36 |
| 310P3C5 | KASEDIYNRLA | 40 | GVTSLET | 41 | QQYWSFPWT | 42 |
| 362H3D3 | RASENIYSNLA | 46 | AATNLAD | 47 | QHFWGTPYT | 48 |

***Table 3. Sequences of DLL3 chimeric mAbs***

| Name | Sequence | SEQ ID NO: |
|---|---|---|
| 9E8D8-hIgG1 Heavy Chain | QVQLQQSGAELVRPGASVKLSCKALGYTFTDSEIHWVKQTPVHGLE WIGAIHPRNGGTAYNQKFRGKATLSADRSSNTAYMELSSLTSEDSA VYYCSRGYFLDYWGQGTSVTVSS<br>ASTKGPSVFPLAPSSKSTSGGTAALGCLVKDYFPEPVTVSWNSGAL TSGVHTFPAVLQSSGLYSLSSVVTVPSSSLGTQTYICNVNHKPSNT KVDKKVEPKSCDKTHTCPPCPAPELLGGPSVFLFPPKPKDTLMISR TPEVTCVVVDVSHEDPEVKFNWYVDGVEVHNAKTKPREEQYNSTYR VVSVLTVLHQDWLNGKEYKCKVSNKALPAPIEKTISKAKGQPREPQ VYTLPPSREEMTKNQVSLTCLVKGFYPSDIAVEWESNGQPENNYKT TPPVLDSDGSFFLYSKLTVDKSRWQQGNVFSCSVMHEALHNHYTQK SLSLSPGK | 49 |
| 9E8D8-hIgG 1 Light Chain | DIVMTQSPSSLTMSVGQKVTMSCKSSQSLLNSDNQKNYLAWYQQRP GQSPKLLLYFASTRESGVPDRFIGSGSGTDFTLTISSVQAEDLADY FCQHHYDTPPTFGGGTKLEIK<br>RTVAAPSVFIFPPSDEQLKSGTASVVCLLNNFYPREAKVQWKVDNA LQSGNSQESVTEQDSKDSTYSLSSTLTLSKADYEKHKVYACEVTHQ GLSSPVTKSFNRGEC | 50 |
| 36B7F3-hIgG1 Heavy Chain | QVQLQQSGPELVKPGASVKISCRASGYTFTDHHLNWVKQRPGQGLE WIGWIFPGSGSDYYNERFKDKATLTVDKSSSTAYMLLSSLTSEDSA VYFCERWDYWGQGTTLTVSS<br>ASTKGPSVFPLAPSSKSTSGGTAALGCLVKDYFPEPVTVSWNSGAL TSGVHTFPAVLQSSGLYSLSSVVTVPSSSLGTQTYICNVNHKPSNT KVDKKVEPKSCDKTHTCPPCPAPELLGGPSVFLFPPKPKDTLMISR TPEVTCVVVDVSHEDPEVKFNWYVDGVEVHNAKTKPREEQYNSTYR VVSVLTVLHQDWLNGKEYKCKVSNKALPAPIEKTISKAKGQPREPQ VYTLPPSREEMTKNQVSLTCLVKGFYPSDIAVEWESNGQPENNYKT TPPVLDSDGSFFLYSKLTVDKSRWQQGNVFSCSVMHEALHNHYTQK SLSLSPGK | 51 |
| 36B7F3-hIgG1 Light Chain | QIVLTQSPTIMSASPGEKVTMTCSASSSISYMHWYQQKPGTSPKRW IYDTSTLASGVPARFSGGGSGTSYSLTISSMEAEDAATYYCHQRSS YPLTFGAGTKLELK<br>RTVAAPSVFIFPPSDEQLKSGTASVVCLLNNFYPREAKVQWKVDNA LQSGNSQESVTEQDSKDSTYSLSSTLTLSKADYEKHKVYACEVTHQ GLSSPVTKSFNRGEC | 52 |

(continued)

| Name | Sequence | SEQ ID NO: |
|---|---|---|
| 129H2B9-hIgG1 Heavy Chain | EVNLEESGGGLVQPGGSMKLSCVVSGFTFSNYWMSWVRQSPEKGLE WVAEIRLKSDNYATHYAESVKGKFTISRDDSKSRLYLQMNSLRPED TGIYYCTSNRFAYWGQGTLVTVSA<br><br>ASTKGPSVFPLAPSSKSTSGGTAALGCLVKDYFPEPVTVSWNSGAL TSGVHTFPAVLQSSGLYSLSSVVTVPSSSLGTQTYICNVNHKPSNT KVDKKVEPKSCDKTHTCPPCPAPELLGGPSVFLFPPKPKDTLMISR TPEVTCVVVDVSHEDPEVKFNWYVDGVEVHNAKTKPREEQYNSTYR VVSVLTVLHQDWLNGKEYKCKVSNKALPAPIEKTISKAKGQPREPQ VYTLPPSREEMTKNQVSLTCLVKGFYPSDIAVEWESNGQPENNYKT TPPVLDSDGSFFLYSKLTVDKSRWQQGNVFSCSVMHEALHNHYTQK SLSLSPGK | 53 |
| 129H2B9-hIgG1 Light Chain | NIVMTQSPKSVSMSVGERVTLSCKASENVGTYVYWYQQKPEQSPKM MIYGASNRYTGVPDRFTGSGSATDFTLTIRSVQAEDLADYHCGQSY SYPFTFGSGTKLEIK<br><br>RTVAAPSVFIFPPSDEQLKSGTASVVCLLNNFYPREAKVQWKVDNA LQSGNSQESVTEQDSKDSTYSLSSTLTLSKADYEKHKVYACEVTHQ GLSSPVTKSFNRGEC | 54 |
| 148C3A7-hIgG1 Heavy Chain | EVQLQQSGPELIKPGTSVKMSCKASGYTFTTYLMHWVKQKPGQGLE WIGYINPYNDVTEYHEKFKDKATLTSDKSSSTAYMELSSLTSEDSA VYYCARLGGYGPGWYLDVWGAGTTVTVSS<br><br>ASTKGPSVFPLAPSSKSTSGGTAALGCLVKDYFPEPVTVSWNSGAL TSGVHTFPAVLQSSGLYSLSSVVTVPSSSLGTQTYICNVNHKPSNT KVDKKVEPKSCDKTHTCPPCPAPELLGGPSVFLFPPKPKDTLMISR TPEVTCVVVDVSHEDPEVKFNWYVDGVEVHNAKTKPREEQYNSTYR VVSVLTVLHQDWLNGKEYKCKVSNKALPAPIEKTISKAKGQPREPQ VYTLPPSREEMTKNQVSLTCLVKGFYPSDIAVEWESNGQPENNYKT TPPVLDSDGSFFLYSKLTVDKSRWQQGNVFSCSVMHEALHNHYTQK SLSLSPGK | 55 |
| 148C3A7-hIgG1 Light Chain | DIQMTQSPASLSASVGETVTITCRASGSIHNYLAWYLHKQGKSPQL LVYNAKTLADGVPSRFSGSGSGTQYSLKINSLQPEDFGSYYCHHFW STPWTFGGGTKLEIK<br><br>RTVAAPSVFIFPPSDEQLKSGTASVVCLLNNFYPREAKVQWKVDNA LQSGNSQESVTEQDSKDSTYSLSSTLTLSKADYEKHKVYACEVTHQ GLSSPVTKSFNRGEC | 56 |
| 310P3C5-hIgG1 Heavy chain | EVQLQQSGPELVKPGASVKISCKASGYTFTDFYMNWVKQTHGKSLE WIGDANPNNGGTTHNPRFKGQATLTVDKSSNTAYMELRSLTSEDSA VYYCARGGLPGDFDYWGQGTTLTVSS<br><br>ASTKGPSVFPLAPSSKSTSGGTAALGCLVKDYFPEPVTVSWNSGAL TSGVHTFPAVLQSSGLYSLSSVVTVPSSSLGTQTYICNVNHKPSNT KVDKKVEPKSCDKTHTCPPCPAPELLGGPSVFLFPPKPKDTLMISR TPEVTCVVVDVSHEDPEVKFNWYVDGVEVHNAKTKPREEQYNSTYR VVSVLTVLHQDWLNGKEYKCKVSNKALPAPIEKTISKAKGQPREPQ VYTLPPSREEMTKNQVSLTCLVKGFYPSDIAVEWESNGQPENNYKT TPPVLDSDGSFFLYSKLTVDKSRWQQGNVFSCSVMHEALHNHYTQK SLSLSPGK | 57 |

(continued)

| Name | Sequence | SEQ ID NO: |
|---|---|---|
| 310P3C5-hIgG1 Light chain | DIQMTQSSSSFSVSLGDRVTITCKASEDIYNRLAWYQQKPGNAPRL LISGVTSLETGVPSRFSGSGSGKDYTLSITSLQTEDVATYYCQQYW SFPWTFGGGTKLEIK RTVAAPSVFIFPPSDEQLKSGTASVVCLLNNFYPREAKVQWKVDNA LQSGNSQESVTEQDSKDSTYSLSSTLTLSKADYEKHKVYACEVTHQ GLSSPVTKSFNRGEC | 58 |
| 362H3D3-hIgG1 Heavy chain | EVQLQQSGPELLKPGASVKISCKASGYTFTDYTMHWVKQSHGRSLE WIGGIHPNYGGTSYNEKFKDKATLTVDKSSSTAYMEFRSLTSEDSA VYYCARWGYYGGSYWYFDVWGTGTTVTVSS ASTKGPSVFPLAPSSKSTSGGTAALGCLVKDYFPEPVTVSWNSGAL TSGVHTFPAVLQSSGLYSLSSVVTVPSSSLGTQTYICNVNHKPSNT KVDKKVEPKSCDKTHTCPPCPAPELLGGPSVFLFPPKPKDTLMISR TPEVTCVVVDVSHEDPEVKFNWYVDGVEVHNAKTKPREEQYNSTYR VVSVLTVLHQDWLNGKEYKCKVSNKALPAPIEKTISKAKGQPREPQ VYTLPPSREEMTKNQVSLTCLVKGFYPSDIAVEWESNGQPENNYKT TPPVLDSDGSFFLYSKLTVDKSRWQQGNVFSCSVMHEALHNHYTQK SLSLSPGK | 59 |
| 362H3D3-hIgG1 Light chain | DIQMTQSPASLSVSVGETVTITCRASENIYSNLAWYQQKQGKSPQL LVYAATNLADGVPSRFSGSGSGTQYSLKINSLQSEDFGSYYCQHFW GTPYTFGGGTKLEIK RTVAAPSVFIFPPSDEQLKSGTASVVCLLNNFYPREAKVQWKVDNA LQSGNSQESVTEQDSKDSTYSLSSTLTLSKADYEKHKVYACEVTHQ GLSSPVTKSFNRGEC | 60 |

**Example 2. Protein Binding Activity of the Chimeric Monoclonal Antibodies Targeting DLL3**

***2.1. ELISA binding to human DLL3***

In order to determine the binding ability of the chimeric mAbs to human DLL3 protein, ELISA-based binding assay was performed as followed. In brief, hDLL3-his protein was diluted with DPBS buffer at 1 μg/mL and adsorbed to wells of 96-well microplates overnight at 4 °C. After blocking the wells with 1% bovine serum albumin (BSA) to prevent non-specific binding, the DLL3 chimeric Abs, benchmark antibodies DLL3-4-001(CC) and DLL3#3, or an isotype control were titrated at a 3-fold dilution rate starting from 100 nM and added to the wells pre-adsorbed with hDLL3-his protein. The mixture was incubated for 1 hour at room temperature (RT). The bound DLL3 mAbs were recognized by a detective antibody against human IgG Fc which was conjugated with horseradish peroxidase (HRP) (Jackson Immuno, Cat. No. 109-035-008). Tetramethylbenzidine (TMB), a substrate of HRP, was added to the wells to visualize the binding signal. After sufficient color development, stop solution was added to the wells. The absorbance of the signal was detected at 450 nm with Envision multilabel plate readers (PerkinElemer). The graph and statistics analysis were generated with four-parameter nonlinear regression curve fit in Graphpad Prism 9 software.

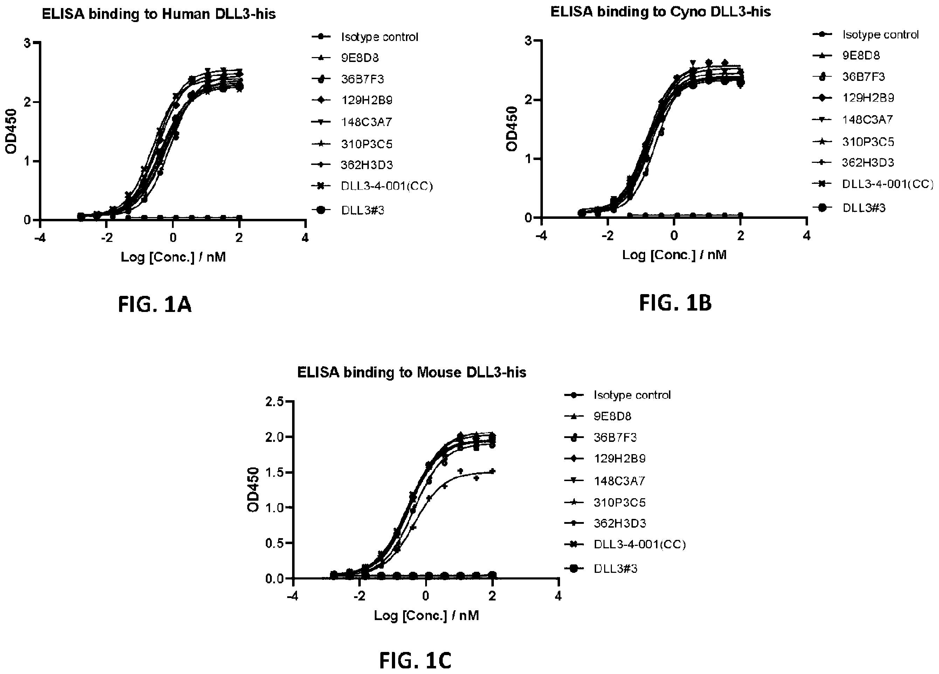

As shown in **FIG. 1A,** all the DLL3 chimeric Abs efficiently bound to human DLL3 protein. The EC50 values of the binding curve of each antibody were summarized in **Table 4.**

***2.2 ELISA binding to cynomolgus and mouse DLL3 protein***

In order to determine the cross reactivity of the chimeric Abs to cynomolgus and mouse DLL3, ELISA binding assay was performed as described above. Recombinant cynoDLL3-his (KACTUS, Cat. No. DLL-CM103) and mouse DLL3 his tag protein (mDLL3-his, KACTUS, Cat. No. DLL-MM103) were used as the coating antigens at 1 μg/mL.

As shown in **FIG. 1B** and **1C,** all the DLL3 chimeric Abs efficiently bound to cynomolgus DLL3 and mouse DLL3 protein.

The EC50 values of the binding curve of each antibody were summarized in **Table 4.**

*2.3. ELISA binding to human DLL1 and DLL4*

Due to high sequence similarities of the family member proteins DLL1 and DLL4 to DLL3, it is necessary to identify the specificity of DLL3 chimeric antibodies.

In order to determine the binding ability of the chimeric Abs to human DLL1 and DLL4 protein, ELISA binding assay was performed as previously described. The hDLL1-his protein (Sino Biological, Inc., Cat. No. 11635-H08H) and hDLL4-his protein (Sino Biological, Inc., Cat. No. 10171-H08H) were used as the coating antigen at 1 μg/mL. DLL1 mAb pidilizumab (CAS No. 1036730-42-3) and DLL4 mAb (Fab) MLCK-2 (Patent No. WO2015005632, ABL Bio) were employed as the positive control.

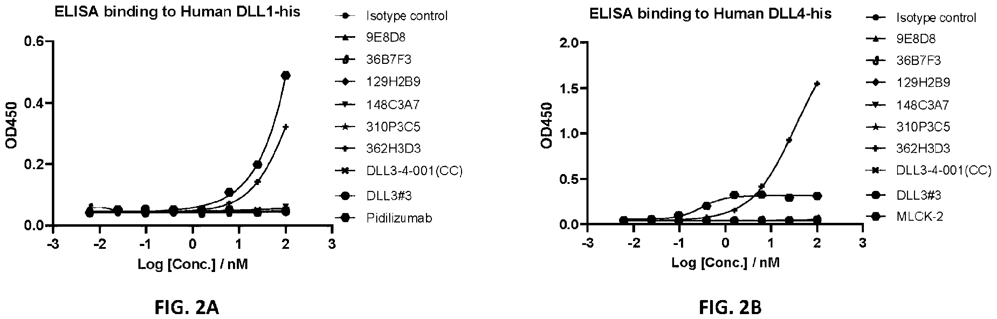

As shown in **FIG. 2A** and **2B,** most of the DLL3 chimeric Abs displayed neglectable binding of either hDLL1 protein or hDLL4 protein, similar to the benchmark antibodies. However, 362H3D3 showed detectable binding to human DLL1 and DLL4 protein, which might represent the cross-reactivity of this DLL3 Ab to other two family members.

***Table 4. Binding activities of the DLL3 antibodies to antigen protein***

| Antigen / Antibody | Human DLL3 | Cynomolgus DLL3 | Mouse DLL3 | Human DLL1 | Human DLL4 |
|---|---|---|---|---|---|
| 9E8D8 | 0.637 | 0.204 | 0.370 | -- | -- |
| 36B7F3 | 0.836 | 0.272 | 0.437 | -- | -- |
| 129H2B9 | 0.364 | 0.149 | 0.332 | -- | -- |
| 148C3A7 | 0.334 | 0.188 | 0.311 | -- | -- |
| 310P3C5 | 0.519 | 0.158 | 0.278 | -- | -- |
| 362H3D3 | 0.482 | 0.160 | 0.447 | + | + |
| DLL3-4-01(CC) | 0.247 | 0.150 | 0.281 | -- | -- |
| DLL3#3 | 0.419 | 0.182 | -- | -- | -- |

--: No binding

*2.4. Affinity measurement*

The binding affinity of the chimeric antibodies to human DLL3 protein was determined with Biacroe™ 8K. Briefly, the antibodies (1 or 2 ug/ml) were captured with a Pro-A chip. Single dose (50 or 100nM) of human DLL3-his protein were injected over captured antibody at a flow rate of 30 μL/min. The antigen was allowed to associate for 120s and dissociate for 150-400s. Data analysis was carried out using Biacore™ 8K evaluation software. The results showed that all the chimeric DLL3 chimeric antibodies exhibited high affinity to human DLL3, which was similar to benchmark antibodies (**Table 5**).

***Table 5. Affinity ranking results of chimeric antibodies by Biacroe™***

| Affinity / Antibody | Human DLL3-his | | |
|---|---|---|---|
| | Ka(1/Ms) | Kd(1/s) | KD(M) |
| 9E8D8 | 5.97E+05 | 2.48E-04 | 4.15E-10 |
| 36B7F3 | 1.06E+06 | 3.21E-05 | 3.04E-11 |
| 129H2B9 | 8.16E+06 | 1.75E-04 | 2.15E-10 |
| 148C3A7 | 8.57E+06 | 1.68E-04 | 1.96E-10 |
| 310P3C5 | 9.71E+05 | 3.96E-04 | 4.08E-10 |
| 362H3D3 | 1.97E+06 | 7.44E-04 | 3.77E-10 |

*2.5. Domain mapping of the chimeric DLL3 antibodies*

In order to evaluate the exact binding domain of the chimeric mAbs to human DLL3, cell based binding assay was employed as followed. Briefly, HEK293 cells stably expressing a series of overlapping ECDs of human DLL3 was used in these assays. The human DLL3 protein (UniProt NO. Q9NYJ7, A27-K618) has a distinct extracellular structure(A27-L492) that consists of one DSL domain, six EGF like repeats and an unstructured membrane proximal extracellular region (MPER) adjacent to the transmembrane domain (TM). Consequently, cell lines expressing EGF1 and EGF2 (EGF1+2, A216-E310), EGF2 and EGF3 (EGF2+3, G274-E351), EGF3 and EGF4 (EGF3+4, S312-E389), EGF4 and EGF5

(EGF4+5, R353-R427), EGF5 and EGF6 (EGF5+6, D393-E465) and EGF6 and MPER (EGF6-MPER, R429-L492) conjugated with the TM and ICD of DLL3 were constructed (customized by Genomeditech). The indicated DLL3 chimeric Abs, the benchmark antibodies, or an isotype control were diluted at 100 nM in the staining buffer (PBS buffer containing 2% FBS) and incubated with $5x10^4$ indicated cells in the 96-well microplate for 30 mins at 4 °C. The antibodies binding to the antigen on the cell surface were detected with Goat anti-Human IgG (H+L) Cross-Adsorbed Secondary Antibody, Alexa Fluor™ 488 (ThermoFisher scientific, Cat. No. A-11013) at a dilution rate of 1:2000. Cells were analyzed by MACSQuant® Analyzer 16 Flow Cytometer (Miltenyi Biotec B.V. & Co. KG). Data were analyzed with Flowjo 10.0 software. The graph and statistics analysis were generated with four-parameter nonlinear regression curve fit in Graphpad Prism 9 software.

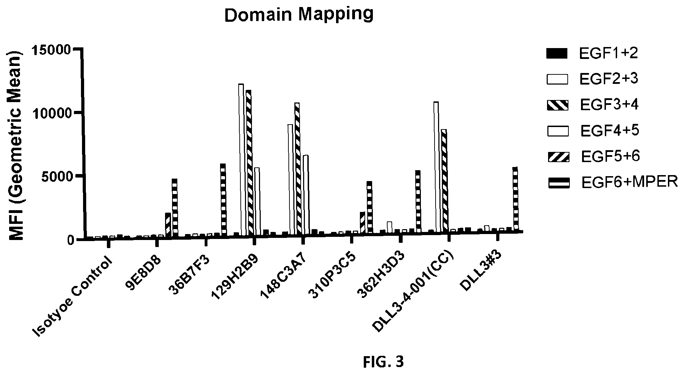

As shown in **FIG. 3** and **Table 6,** these DLL3 chimeric antibodies bound to different ECD domains of human DLL3 expressed on HEK293 cells. More specifically, 129H2B9 and 148C3A7 bound to the EGF3-4 domain of human DLL3 expressed on cells. 9E8D8 and 310P3C5 specifically bound to the EGF6 domain of human DLL3 expressed on cells. 36B7and 362H3D3 bound to the membrane proximal extracellular domain which is similar to the benchmark antibody DLL3#3. The other benchmark antibody DLL3-4-001(CC) specifically bound to EGF3 which was consistent with previously reported data.

***Table 6. Domain binding properties of chimeric antibodies***

| Antibody / Domain | MFI (Geometric Mean, 100nM) | | | | | | | | |
|---|---|---|---|---|---|---|---|---|---|
| | **Isotype** | **9E8D8** | **36B7F3** | **129H2B9** | **148C3A7** | **310P3C5** | **362H3D3** | **DLL3-4-001(CC)** | **DLL3#3** |
| **EGF1+2** | 237 | 241 | 274 | 348 | 337 | 241 | 337 | 264 | 268 |
| **EGF2+3** | 242 | 241 | 301 | **12022** | **8763** | 267 | 976 | **10338** | 528 |
| **EGF3+4** | 269 | 286 | 258 | **11514** | **10464** | 289 | 352 | **8174** | 310 |
| **EGF4+5** | 270 | 269 | 288 | **5418** | **6314** | 307 | 337 | 322 | 308 |
| **EGF5+6** | 329 | **1980** | 349 | 515 | 466 | **1782** | 390 | 362 | 366 |
| **EGF6 +MPER** | 249 | **4654** | **5774** | 302 | 313 | **4178** | **4951** | 409 | **5105** |
| **Domain** | / | **EGF6** | **MPER** | **EGF3-4** | **EGF3-4** | **EGF6** | **EGF7** | **EGF3** | **MPER** |

**Example 3. Cell Binding Activity of the Chimeric Monoclonal Antibodies Targeting DLL3**

***3.1 Binding ability to human DLL3 over-expressed on HEK-293 cells***

In order to evaluate the binding activity of the chimeric mAbs to human DLL3 expressed on cells, cell based binding assay was employed as followed. Briefly, HEK293 cells stably expressing high level of ECD of human DLL3 (HEK293-hDLL3) was used in these assays. The indicated DLL3 chimeric Abs, the benchmark antibodies, or an isotype control were diluted at a three-fold dilution rate starting from a concentration of 50 nM in the staining buffer (PBS buffer containing 2% FBS). The antibody dilutions were incubated with $5x10^4$ indicated cells in the 96-well microplate for 30 mins at 4 °C. The antibodies binding to the antigen on the cell surface were detected with Goat anti-Human IgG (H+L) Cross-Adsorbed Secondary Antibody, Alexa Fluor™ 488 (ThermoFisher scientific, Cat. No. A-11013) at a dilution rate of 1:2000. Cells were analyzed by MACSQuant® Analyzer 16 Flow Cytometer (Miltenyi Biotec B.V. & Co. KG). Data were analyzed with Flowjo 10.0 software. The graph and statistics analysis were generated with four-parameter nonlinear regression curve fit in Graphpad Prism 9 software.

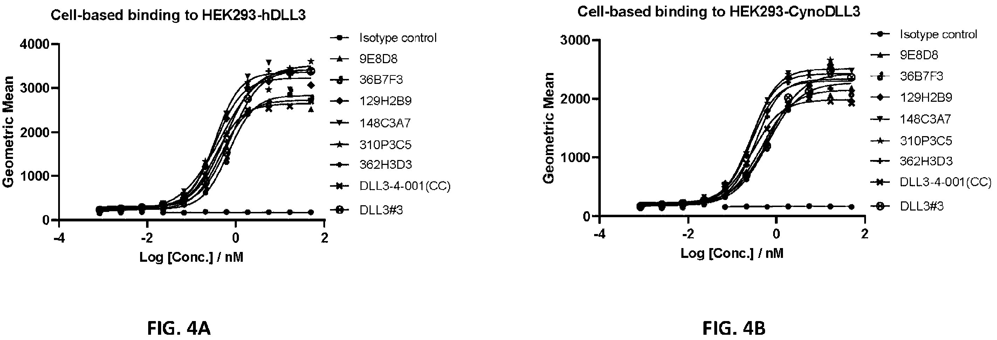

As shown in **FIG. 4A,** all the DLL3 chimeric antibodies efficiently bound to human DLL3 expressed on HEK293 cells in a dose-dependent manner. Of note, chimeric antibodies 129H2B9, 148C3A7, 310P3C5 and 362H3D3 exhibited superior binding potency to human DLL3 expressed on HEK293 cells when compared with the benchmark antibodies DLL3-4-001(CC) and DLL3#3 as indicated by either improved EC50 vs. DLL3-4-001(CC) or improved maximal binding signal vs. DLL3#3. 9E8D8 and 36B7F3 exhibited comparable maximum binding potency to human DLL3 expressed on HEK293 cells with that of EGF3 binding benchmark DLL3-4-001(CC).

The EC50 values of the binding capability to human DLL3 expressed on cells were summarized in **Table 7.**

***3.2 Binding ability to cyno DLL3 over-expressed on cells***

In order to evaluate species cross-reactivity of the chimeric mAbs to cynomolgus DLL3 expressed on cells, cell

based binding assay was employed following the protocol described before. Briefly, HEK293 cells stably expressing human full length cyno DLL3 (HEK293-cynoDLL3) were constructed (customized by Cusabio). The indicated DLL3 chimeric Abs, the benchmark antibodies, or an isotype control were tested in this assay.

As shown in **FIG. 4B,** all the DLL3 chimeric antibodies efficiently bound to cyno DLL3 expressed on HEK293 cells with comparable binding potency to their binding to human DLL3.

The EC50 of the binding capability to cynomolgus DLL3 expressed on cells were summarized in **Table 7.**

***3.3 Binding to human DLL3 expressed on tumor cells***

In order to evaluate the binding activity of the chimeric mAbs to human DLL3 expressed on tumor cells, human SCLC cell lines SHP77 and NCI-H82 with low level of DLL3 expression were employed in the cell-based binding assay following the protocol described in **Example 3.1.**

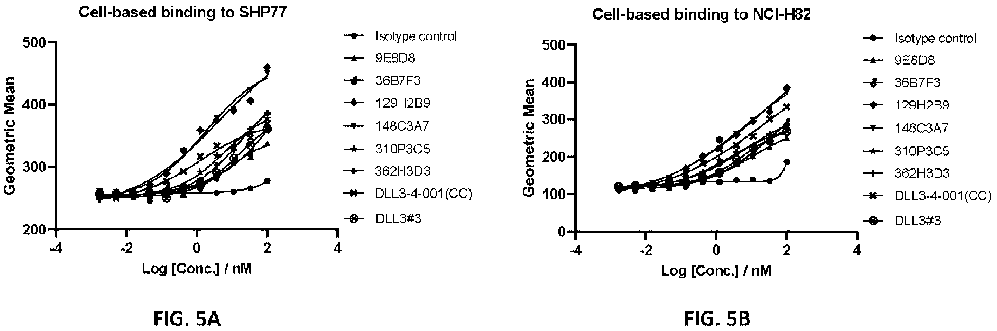

As shown in **FIG. 5A, 5B,** all the DLL3 chimeric antibodies efficiently bound to human DLL3 expressed on SCLC tumor cell line SHP77 or NCI-H82. Interestingly, different from the binding trends on DLL3 overexpressing HEK293 cells, DLL3 mAbs 129H2B9 and 148C3A7 binding to the membrane distal region showed significantly better binding potency than membrane proximal binding mAbs including 9E8D8, 36B7F3, 310P3C5 and 362H3D3 in these two DLL3 expressing tumor cell lines, indicating the superiority of the binding capability of membrane distal binders (**FIG. 5A** and **5B**).

***3.4 Binding ability to human DLL1 and DLL4 over-expressed on cells***

In order to exclude non-specific binding of the chimeric mAbs to human DLL1 and DLL4 expressed on cells, cell based binding assay was employed as described before. Briefly, CHO-K1 cells stably expressing human full length DLL1 and DLL4 (CHO-K1-hDLL1 and CHO-K1-hDLL4, Genomeditech) were constructed. The sequences of human DLL1 and DLL4 used in the present Example are derived from UniProt (O00548 and Q9NR61). Protein expression was confirmed by FACS analyses with anti-human DLL1 (R&D Systems, MAB1818) and anti-human DLL4 (R&D system, MAB1506) antibodies. The indicated DLL3 chimeric Abs, the benchmark antibodies, or an isotype control were tested in the assays.

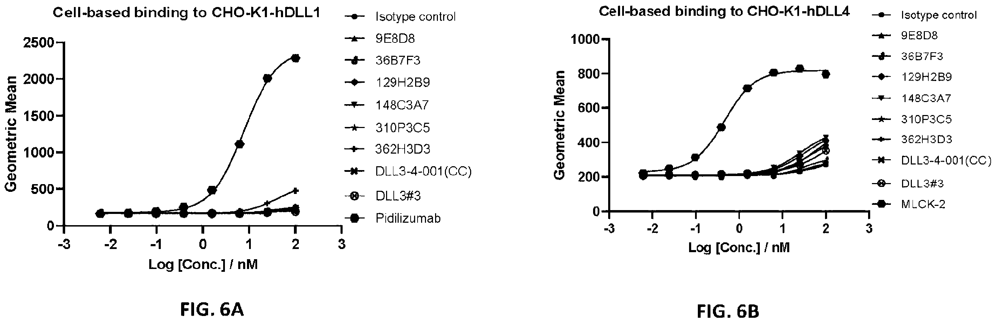

As shown in **FIG. 6A,** in contrast to the significant binding ability of the positive control DLL1 mAb pidilizumab to DLL1, no specific binding was detected to human DLL1 expressed on CHO-K1 cells in most of the DLL3 chimeric antibodies including 9E8D8, 36B7F3, 129H2B9, 148C3A7, 310P3C5 and two benchmark antibodies. 362H3D3 showed a subtle binding to DLL1 overexpressed on CHO-K1 cells only at the highest dose level 100 nM. Moreover, similar to two benchmark antibodies DLL3-4-01(CC) and DLL3#3, all the DLL3 chimeric antibodies showed negligible binding to DLL4 overexpressed on CHO-K1 cells, which is contrary to the significant binding intensity of DLL4 specific positive control antibody MLCK-2 (**FIG. 6B**). Overall, all the chimeric antibodies exhibited high specificityin binding to human DLL3 expressed on cells.

***Table 7. Binding properties of chimeric antibodies to different antigens***

| EC50(nM) / Antibody | HEK293-hDLL3 | HEK293-cynoDLL3 | HEK293-hDLL1 | HEK293-hDLL4 |
|---|---|---|---|---|
| 9E8D8 | 0.459 | 0.446 | -- | -- |
| 36B7F3 | 0.686 | 0.616 | -- | -- |
| 129H2B9 | 0.343 | 0.259 | -- | -- |
| 148C3A7 | 0.346 | 0.279 | -- | -- |
| 310P3C5 | 0.455 | 0.257 | -- | -- |
| 362H3D3 | 0.558 | 0.335 | -- | -- |
| DLL3-4-01(CC) | 0.339 | 0.277 | -- | -- |
| DLL3#3 | 0.760 | 0.658 | -- | -- |

--: No binding

**Example 4. Humanization of the chimeric DLL3 antibodies**

The variable regions of 310P3C5 chimeric antibodies were selected to perform humanization.

Briefly, the amino acid sequences of the VH and VL were aligned with the available database of human Ig gene

sequences to identify the overall best-matching human germline Ig gene sequences. Then, the CDRs of heavy chain and light chain of the chimeric antibody were grafted onto the candidate human germlines. A 3D model of the CDR grafted antibody was generated by Molecular Operating Environment (MOE) to determine if there was any critical human amino acid in the framework region essential to be back-mutated to corresponding mouse amino acids to maintain the CDR conformation and antibody function.

For the heavy chain of 310P3C5, the candidate human germline sequences were the *IGKV1-18*01* or *IGKV1-3*01* gene. For the light chain of 310P3C5, the candidate human germline sequences were the *IGKVI-12*01* or *IGKV1-33*01* gene. In the case of the heavy chain, M48I, R67Q, V68A, M70L, T72V and T74K in the framework of *IGKV1-18*01,* and R38K, M48I, V68A, 170L, R72V, T74K and S84R in the framework of *IGKVI-3*01* were involved in back-mutations. In the case of the light chain, Y49S, Y49S and T69K in the framework regions of IGKV1-33*01 and IGKV1-12*01, separately, were involved in back-mutations.

Different combinations of backmutation sites were selected to generate variable regions of humanized antibodies. The sequences of the variable regions of the heavy chain and light chain of the humanized antibodies for 310P3C5 were listed in **Table 8A.** The pairing of the VH and VL for individual humanized antibodies was listed in **Table 8B.** The variable regions of humanized antibodies were then fused to the constant region of human IgG1 for antibody production and functional characterization. The full sequences of the heavy chain and light chain of the humanized antibodies were listed in **Table 8C.**

***Table 8A. The Sequences of variable region of humanized antibodies (underlining/bold indicates CDR; bold/italic indicates back mutations)***

| **310P3C5** | | |
|---|---|---|
| | **Sequence** | **SEQ NO ID:** |
| 310P3C5 VH | EVQLQQSGPELVKPGASVKISCKASGYTFT**DFYMN**WVKQTHGKSLEWIG **DANPNNGGTTHNPRFKG**QATLTVDKSSNTAYMELRSLTSEDSAVYYCAR **GGLPGDFDY**WGQGTTLTVSS | 9 |
| 310P3C5 HU-VH1-1 | QVQLVQSGAEVKKPGASVKVSCKASGYTFT**DFYMN**WVRQAPGQGLEWMG **DANPNNGGTTHNPRFKG**RVTMTTDTSTSTAYMELRSLRSDDTAVYYCAR **GGLPGDFDY**WGQGTTVTVSS | 65 |
| 310P3C5 HU-VH1-2 | QVQLVQSGAEVKKPGASVKVSCKASGYTFT**DFYMN**WVRQAPGQGLEW***I***G **DANPNNGGTTHNPRFKG**R***A***TMTTDTSTSTAYMELRSLRSDDTAVYYCAR **GGLPGDFDY**WGQGTTVTVSS | 66 |
| 310P3C5 HU-VH1-3 | QVQLVQSGAEVKKPGASVKVSCKASGYTFT**DFYMN**WVRQAPGQGLEW***I***G **DANPNNGGTTHNPRFKG**R***A***T***L***T***V***DTSTSTAYMELRSLRSDDTAVYYCAR **GGLPGDFDY**WGQGTTVTVSS | 67 |
| 310P3C5 HU-VH1-4 | QVQLVQSGAEVKKPGASVKVSCKASGYTFT**DFYMN**WVRQAPGQGLEW***I***G **DANPNNGGTTHNPRFKG*****QA***T***L***T***V***D***K***STSTAYMELRSLRSDDTAVYYCAR **GGLPGDFDY**WGQGTTVTVSS | 68 |
| IGKV1-18*01 | QVQLVQSGAEVKKPGASVKVSCKASGYTFTSYGISWVRQAPGQGLEWMG WISAYNGNTNYAQKLQGRVTMTTDTSTSTAYMELRSLRSDDTAVYYCAR | 61 |
| 310P3C5 HU-VH2 | QVQLVQSGAEVKKPGASVKVSCKASGYTFT**DFYMN**WV***K***QAPGQGLEW***I***G **DANPNNGGTTHNPRFKG**R***A***T***L***T***V***D***K***SASTAYMEL***R***SLRSEDTAVYYCAR **GGLPGDFDY**WGQGTTVTVSS | 69 |
| IGKV1-3*01 | QVQLVQSGAEVKKPGASVKVSCKASGYTFTSYAMHWVRQAPGQRLEWMG WINAGNGNTKYSQKFQGRVTITRDTSASTAYMELSSLRSEDTAVYYCAR | 62 |
| 310P3C5 VL | DIQMTQSSSSFSVSLGDRVTITC**KASEDIYNRLA**WYQQKPGNAPRLLIS **GVTSLET**GVPSRFSGSGSGKDYTLSITSLQTEDVATYYC**QQYWSFPWT**F GGGTKLEIK | 10 |
| 310P3C5 HU-VLI-1 | DIQMTQSPSSVSASVGDRVTITC**KASEDIYNRLA**WYQQKPGKAPKLLIY **GVTSLET**GVPSRFSGSGSGTDFTLTISSLQPEDFATYYC**QQYWSFPWT**F GGGTKVEIK | 70 |

(continued)

| 310P3C5 | | |
|---|---|---|
| | **Sequence** | **SEQ NO ID:** |
| 310P3C5 HU-VL1-2 | DIQMTQSPSSVSASVGDRVTITC**KASEDIYNRLA**WYQQKPGKAPKLLI***S***<br>**GVTSLET**GVPSRFSGSGSGTDFTLTISSLQPEDFATYYC**QQYWSFPWT**F<br>GGGTKVEIK | 71 |
| 310P3C5 HU-VL1-3 | DIQMTQSPSSVSASVGDRVTITC**KASEDIYNRLA**WYQQKPGKAPKLLI***S***<br>**GVTSLET**GVPSRFSGSGSG***K***DFTLTISSLQPEDFATYYC**QQYWSFPWT**F<br>GGGTKVEIK | 72 |
| IGKV1-12*01 | DIQMTQSPSSVSASVGDRVTITCRASQGISSWLAWYQQKPGKAPKLLIY<br>AASSLQSGVPSRFSGSGSGTDFTLTISSLQPEDFATYYCQQANSFP | 63 |
| 310P3C5 HU-VL2 | DIQMTQSPSSLSASVGDRVTITC**KASEDIYNRLA**WYQQKPGKAPKLLI***S***<br>**GVTSLET**GVPSRFSGSGSGTDFTFTISSLQPEDIATYYC**QQYWSFPWT**F<br>GGGTKVEIK | 73 |
| IGKV1-33*01 | DIQMTQSPSSLSASVGDRVTITCQASQDISNYLNWYQQKPGKAPKLLIY<br>DASNLETGVPSRFSGSGSGTDFTFTISSLQPEDIATYYCQQYDNLP | 64 |

***Table 8B. Pairing of VH and VL for humanized antibodies***

| ***310P3C5*** | **HU-VL1-1** | **HU-VL1-2** | **HU-VL1-3** | **HU-VL2** | **VL** |
|---|---|---|---|---|---|
| **HU-VH1-1** | z1 | z5 | z9 | z13 | |
| **HU-VH1-2** | z2 | z6 | z10 | | |
| **HU-VH1-3** | z3 | z7 | z11 | | |
| **HU-VH1-4** | z4 | z8 | z12 | | |
| **HU-VH2** | z14 | | | z15 | |
| **VH** | | | | | Chimeric |

*Table 8C. Sequences of the heavy chain and light chain of the humanized antibodies*

| 310P3C5 | | |
|---|---|---|
| | **Sequence** | **SEQ NO ID:** |
| 310P3C5 Heavy chain | EVQLQQSGPELVKPGASVKISCKASGYTFT**DFYMN**WVKQTHGKSLEWIG<br>**DANPNNGGTTHNPRFKG**QATLTVDKSSNTAYMELRSLTSEDSAVYYCAR<br>**GGLPGDFDY**WGQGTTLTVSS<br>ASTKGPSVFPLAPSSKSTSGGTAALGCLVKDYFPEPVTVSWNSGALTSG<br>VHTFPAVLQSSGLYSLSSVVTVPSSSLGTQTYICNVNHKPSNTKVDKKV<br>EPKSCDKTHTCPPCPAPELLGGPSVFLFPPKPKDTLMISRTPEVTCVVV<br>DVSHEDPEVKFNWYVDGVEVHNAKTKPREEQYNSTYRVVSVLTVLHQDW<br>LNGKEYKCKVSNKALPAPIEKTISKAKGQPREPQVYTLPPSREEMTKNQ<br>VSLTCLVKGFYPSDIAVEWESNGQPENNYKTTPPVLDSDGSFFLYSKLT<br>VDKSRWQQGNVFSCSVMHEALHNHYTQKSLSLSPGK | 57 |
| 310P3C5 Light chain | DIQMTQSSSSFSVSLGDRVTITC**KASEDIYNRLA**WYQQKPGNAPRLLIS<br>**GVTSLET**GVPSRFSGSGSGKDYTLSITSLQTEDVATYYC**QQYWSFPWT**F<br>GGGTKLEIK<br>RTVAAPSVFIFPPSDEQLKSGTASVVCLLNNFYPREAKVQWKVDNALQS<br>GNSQESVTEQDSKDSTYSLSSTLTLSKADYEKHKVYACEVTHQGLSSPV<br>TKSFNRGEC | 58 |

(continued)

| 310P3C5 | | |
|---|---|---|
| | **Sequence** | **SEQ NO ID:** |
| 310P3C5-z1 Heavy chain | QVQLVQSGAEVKKPGASVKVSCKASGYTFT**DFYMN**WVRQAPGQGLEWMG **DANPNNGGTTHNPRFKG**RVTMTTDTSTSTAYMELRSLRSDDTAVYYCAR **GGLPGDFDY**WGQGTTVTVSS ASTKGPSVFPLAPSSKSTSGGTAALGCLVKDYFPEPVTVSWNSGALTSG VHTFPAVLQSSGLYSLSSVVTVPSSSLGTQTYICNVNHKPSNTKVDKKV EPKSCDKTHTCPPCPAPELLGGPSVFLFPPKPKDTLMISRTPEVTCVVV DVSHEDPEVKFNWYVDGVEVHNAKTKPREEQYNSTYRVVSVLTVLHQDW LNGKEYKCKVSNKALPAPIEKTISKAKGQPREPQVYTLPPSREEMTKNQ VSLTCLVKGFYPSDIAVEWESNGQPENNYKTTPPVLDSDGSFFLYSKLT VDKSRWQQGNVFSCSVMHEALHNHYTQKSLSLSPGK | 74 |
| 310P3C5-zl Light chain | DIQMTQSPSSVSASVGDRVTITC**KASEDIYNRLA**WYQQKPGKAPKLLIY **GVTSLET**GVPSRFSGSGSGTDFTLTISSLQPEDFATYYC**QQYWSFPWT**F GGGTKVEIK RTVAAPSVFIFPPSDEQLKSGTASVVCLLNNFYPREAKVQWKVDNALQS GNSQESVTEQDSKDSTYSLSSTLTLSKADYEKHKVYACEVTHQGLSSPV TKSFNRGEC | 75 |
| 310P3C5-z2 Heavy chain | QVQLVQSGAEVKKPGASVKVSCKASGYTFT**DFYMN**WVRQAPGQGLEWIG **DANPNNGGTTHNPRFKG**RATMTTDTSTSTAYMELRSLRSDDTAVYYCAR **GGLPGDFDY**WGQGTTVTVSS ASTKGPSVFPLAPSSKSTSGGTAALGCLVKDYFPEPVTVSWNSGALTSG VHTFPAVLQSSGLYSLSSVVTVPSSSLGTQTYICNVNHKPSNTKVDKKV EPKSCDKTHTCPPCPAPELLGGPSVFLFPPKPKDTLMISRTPEVTCVVV DVSHEDPEVKFNWYVDGVEVHNAKTKPREEQYNSTYRVVSVLTVLHQDW LNGKEYKCKVSNKALPAPIEKTISKAKGQPREPQVYTLPPSREEMTKNQ VSLTCLVKGFYPSDIAVEWESNGQPENNYKTTPPVLDSDGSFFLYSKLT VDKSRWQQGNVFSCSVMHEALHNHYTQKSLSLSPGK | 76 |
| 310P3C5-z2 Light chain | DIQMTQSPSSVSASVGDRVTITC**KASEDIYNRLA**WYQQKPGKAPKLLIY **GVTSLET**GVPSRFSGSGSGTDFTLTISSLQPEDFATYYC**QQYWSFPWT**F GGGTKVEIK RTVAAPSVFIFPPSDEQLKSGTASVVCLLNNFYPREAKVQWKVDNALQS GNSQESVTEQDSKDSTYSLSSTLTLSKADYEKHKVYACEVTHQGLSSPV TKSFNRGEC | 77 |
| 310P3C5-z3 Heavy chain | QVQLVQSGAEVKKPGASVKVSCKASGYTFT**DFYMN**WVRQAPGQGLEWIG **DANPNNGGTTHNPRFKG**RATLTVDTSTSTAYMELRSLRSDDTAVYYCAR **GGLPGDFDY**WGQGTTVTVSS ASTKGPSVFPLAPSSKSTSGGTAALGCLVKDYFPEPVTVSWNSGALTSG VHTFPAVLQSSGLYSLSSVVTVPSSSLGTQTYICNVNHKPSNTKVDKKV EPKSCDKTHTCPPCPAPELLGGPSVFLFPPKPKDTLMISRTPEVTCVVV DVSHEDPEVKFNWYVDGVEVHNAKTKPREEQYNSTYRVVSVLTVLHQDW LNGKEYKCKVSNKALPAPIEKTISKAKGQPREPQVYTLPPSREEMTKNQ VSLTCLVKGFYPSDIAVEWESNGQPENNYKTTPPVLDSDGSFFLYSKLT VDKSRWQQGNVFSCSVMHEALHNHYTQKSLSLSPGK | 78 |
| 310P3C5-z3 Light chain | DIQMTQSPSSVSASVGDRVTITC**KASEDIYNRLA**WYQQKPGKAPKLLIY **GVTSLET**GVPSRFSGSGSGTDFTLTISSLQPEDFATYYC**QQYWSFPWT**F GGGTKVEIK RTVAAPSVFIFPPSDEQLKSGTASVVCLLNNFYPREAKVQWKVDNALQS GNSQESVTEQDSKDSTYSLSSTLTLSKADYEKHKVYACEVTHQGLSSPV TKSFNRGEC | 79 |

(continued)

| 310P3C5 | | |
|---|---|---|
| | **Sequence** | **SEQ NO ID:** |
| 310P3C5-z4 Heavy chain | QVQLVQSGAEVKKPGASVKVSCKASGYTFT**DFYMN**WVRQAPGQGLEWIG **DANPNNGGTTHNPRFKG**QATLTVDKSTSTAYMELRSLRSDDTAVYYCAR **GGLPGDFDY**WGQGTTVTVSS ASTKGPSVFPLAPSSKSTSGGTAALGCLVKDYFPEPVTVSWNSGALTSG VHTFPAVLQSSGLYSLSSVVTVPSSSLGTQTYICNVNHKPSNTKVDKKV EPKSCDKTHTCPPCPAPELLGGPSVFLFPPKPKDTLMISRTPEVTCVVV DVSHEDPEVKFNWYVDGVEVHNAKTKPREEQYNSTYRVVSVLTVLHQDW LNGKEYKCKVSNKALPAPIEKTISKAKGQPREPQVYTLPPSREEMTKNQ VSLTCLVKGFYPSDIAVEWESNGQPENNYKTTPPVLDSDGSFFLYSKLT VDKSRWQQGNVFSCSVMHEALHNHYTQKSLSLSPGK | 80 |
| 310P3C5-z4 Light chain | DIQMTQSPSSVSASVGDRVTITC**KASEDIYNRLA**WYQQKPGKAPKLLIY **GVTSLET**GVPSRFSGSGSGTDFTLTISSLQPEDFATYYC**QQYWSFPWT**F GGGTKVEIK RTVAAPSVFIFPPSDEQLKSGTASVVCLLNNFYPREAKVQWKVDNALQS GNSQESVTEQDSKDSTYSLSSTLTLSKADYEKHKVYACEVTHQGLSSPV TKSFNRGEC | 81 |
| 310P3C5-z5 Heavy chain | QVQLVQSGAEVKKPGASVKVSCKASGYTFT**DFYMN**WVRQAPGQGLEWMG **DANPNNGGTTHNPRFKG**RVTMTTDTSTSTAYMELRSLRSDDTAVYYCAR **GGLPGDFDY**WGQGTTVTVSS ASTKGPSVFPLAPSSKSTSGGTAALGCLVKDYFPEPVTVSWNSGALTSG VHTFPAVLQSSGLYSLSSVVTVPSSSLGTQTYICNVNHKPSNTKVDKKV EPKSCDKTHTCPPCPAPELLGGPSVFLFPPKPKDTLMISRTPEVTCVVV DVSHEDPEVKFNWYVDGVEVHNAKTKPREEQYNSTYRVVSVLTVLHQDW LNGKEYKCKVSNKALPAPIEKTISKAKGQPREPQVYTLPPSREEMTKNQ VSLTCLVKGFYPSDIAVEWESNGQPENNYKTTPPVLDSDGSFFLYSKLT VDKSRWQQGNVFSCSVMHEALHNHYTQKSLSLSPGK | 82 |
| 310P3C5-z5 Light chain | DIQMTQSPSSVSASVGDRVTITC**KASEDIYNRLA**WYQQKPGKAPKLLIS **GVTSLET**GVPSRFSGSGSGTDFTLTISSLQPEDFATYYC**QQYWSFPWT**F GGGTKVEIK RTVAAPSVFIFPPSDEQLKSGTASVVCLLNNFYPREAKVQWKVDNALQS GNSQESVTEQDSKDSTYSLSSTLTLSKADYEKHKVYACEVTHQGLSSPV TKSFNRGEC | 83 |
| 310P3C5-z6 Heavy chain | QVQLVQSGAEVKKPGASVKVSCKASGYTFT**DFYMN**WVRQAPGQGLEWIG **DANPNNGGTTHNPRFKG**RATMTTDTSTSTAYMELRSLRSDDTAVYYCAR **GGLPGDFDY**WGQGTTVTVSS ASTKGPSVFPLAPSSKSTSGGTAALGCLVKDYFPEPVTVSWNSGALTSG VHTFPAVLQSSGLYSLSSVVTVPSSSLGTQTYICNVNHKPSNTKVDKKV EPKSCDKTHTCPPCPAPELLGGPSVFLFPPKPKDTLMISRTPEVTCVVV DVSHEDPEVKFNWYVDGVEVHNAKTKPREEQYNSTYRVVSVLTVLHQDW LNGKEYKCKVSNKALPAPIEKTISKAKGQPREPQVYTLPPSREEMTKNQ VSLTCLVKGFYPSDIAVEWESNGQPENNYKTTPPVLDSDGSFFLYSKLT VDKSRWQQGNVFSCSVMHEALHNHYTQKSLSLSPGK | 84 |
| 310P3C5-z6 Light chain | DIQMTQSPSSVSASVGDRVTITC**KASEDIYNRLA**WYQQKPGKAPKLLIS **GVTSLET**GVPSRFSGSGSGTDFTLTISSLQPEDFATYYC**QQYWSFPWT**F GGGTKVEIK RTVAAPSVFIFPPSDEQLKSGTASVVCLLNNFYPREAKVQWKVDNALQS GNSQESVTEQDSKDSTYSLSSTLTLSKADYEKHKVYACEVTHQGLSSPV TKSFNRGEC | 85 |

(continued)

| 310P3C5 | | |
|---|---|---|
| | **Sequence** | **SEQ NO ID:** |
| 310P3C5-z7 Heavy chain | QVQLVQSGAEVKKPGASVKVSCKASGYTFT**DFYMN**WVRQAPGQGLEWIG<br>**DANPNNGGTTHNPRFKG**RATLTVDTSTSTAYMELRSLRSDDTAVYYCAR<br>**GGLPGDFDY**WGQGTTVTVSS<br>ASTKGPSVFPLAPSSKSTSGGTAALGCLVKDYFPEPVTVSWNSGALTSG<br>VHTFPAVLQSSGLYSLSSVVTVPSSSLGTQTYICNVNHKPSNTKVDKKV<br>EPKSCDKTHTCPPCPAPELLGGPSVFLFPPKPKDTLMISRTPEVTCVVV<br>DVSHEDPEVKFNWYVDGVEVHNAKTKPREEQYNSTYRVVSVLTVLHQDW<br>LNGKEYKCKVSNKALPAPIEKTISKAKGQPREPQVYTLPPSREEMTKNQ<br>VSLTCLVKGFYPSDIAVEWESNGQPENNYKTTPPVLDSDGSFFLYSKLT<br>VDKSRWQQGNVFSCSVMHEALHNHYTQKSLSLSPGK | 86 |
| 310P3C5-z7 Light chain | DIQMTQSPSSVSASVGDRVTITC**KASEDIYNRLA**WYQQKPGKAPKLLIS<br>**GVTSLET**GVPSRFSGSGSGTDFTLTISSLQPEDFATYYC**QQYWSFPWT**F<br>GGGTKVEIK<br>RTVAAPSVFIFPPSDEQLKSGTASVVCLLNNFYPREAKVQWKVDNALQS<br>GNSQESVTEQDSKDSTYSLSSTLTLSKADYEKHKVYACEVTHQGLSSPV<br>TKSFNRGEC | 87 |
| 310P3C5-z8 Heavy chain | QVQLVQSGAEVKKPGASVKVSCKASGYTFT**DFYMN**WVRQAPGQGLEWIG<br>**DANPNNGGTTHNPRFKG**QATLTVDKSTSTAYMELRSLRSDDTAVYYCAR<br>**GGLPGDFDY**WGQGTTVTVSS<br>ASTKGPSVFPLAPSSKSTSGGTAALGCLVKDYFPEPVTVSWNSGALTSG<br>VHTFPAVLQSSGLYSLSSVVTVPSSSLGTQTYICNVNHKPSNTKVDKKV<br>EPKSCDKTHTCPPCPAPELLGGPSVFLFPPKPKDTLMISRTPEVTCVVV<br>DVSHEDPEVKFNWYVDGVEVHNAKTKPREEQYNSTYRVVSVLTVLHQDW<br>LNGKEYKCKVSNKALPAPIEKTISKAKGQPREPQVYTLPPSREEMTKNQ<br>VSLTCLVKGFYPSDIAVEWESNGQPENNYKTTPPVLDSDGSFFLYSKLT<br>VDKSRWQQGNVFSCSVMHEALHNHYTQKSLSLSPGK | 88 |
| 310P3C5-z8 Light chain | DIQMTQSPSSVSASVGDRVTITC**KASEDIYNRLA**WYQQKPGKAPKLLIS<br>**GVTSLET**GVPSRFSGSGSGTDFTLTISSLQPEDFATYYC**QQYWSFPWT**F<br>GGGTKVEIK<br>RTVAAPSVFIFPPSDEQLKSGTASVVCLLNNFYPREAKVQWKVDNALQS<br>GNSQESVTEQDSKDSTYSLSSTLTLSKADYEKHKVYACEVTHQGLSSPV<br>TKSFNRGEC | 89 |
| 310P3C5-z9 Heavy chain | QVQLVQSGAEVKKPGASVKVSCKASGYTFT**DFYMN**WVRQAPGQGLEWMG<br>**DANPNNGGTTHNPRFKG**RVTMTTDTSTSTAYMELRSLRSDDTAVYYCAR<br>**GGLPGDFDY**WGQGTTVTVSS<br>ASTKGPSVFPLAPSSKSTSGGTAALGCLVKDYFPEPVTVSWNSGALTSG<br>VHTFPAVLQSSGLYSLSSVVTVPSSSLGTQTYICNVNHKPSNTKVDKKV<br>EPKSCDKTHTCPPCPAPELLGGPSVFLFPPKPKDTLMISRTPEVTCVVV<br>DVSHEDPEVKFNWYVDGVEVHNAKTKPREEQYNSTYRVVSVLTVLHQDW<br>LNGKEYKCKVSNKALPAPIEKTISKAKGQPREPQVYTLPPSREEMTKNQ<br>VSLTCLVKGFYPSDIAVEWESNGQPENNYKTTPPVLDSDGSFFLYSKLT<br>VDKSRWQQGNVFSCSVMHEALHNHYTQKSLSLSPGK | 90 |
| 310P3C5-z9 Light chain | DIQMTQSPSSVSASVGDRVTITC**KASEDIYNRLA**WYQQKPGKAPKLLIS<br>**GVTSLET**GVPSRFSGSGSGKDFTLTISSLQPEDFATYYC**QQYWSFPWT**F<br>GGGTKVEIK<br>RTVAAPSVFIFPPSDEQLKSGTASVVCLLNNFYPREAKVQWKVDNALQS<br>GNSQESVTEQDSKDSTYSLSSTLTLSKADYEKHKVYACEVTHQGLSSPV<br>TKSFNRGEC | 91 |

(continued)

| 310P3C5 | | |
|---|---|---|
| | **Sequence** | **SEQ NO ID:** |
| 310P3C5-z10 Heavy chain | QVQLVQSGAEVKKPGASVKVSCKASGYTFT**DFYMN**WVRQAPGQGLEWIG **DANPNNGGTTHNPRFKG**RATMTTDTSTSTAYMELRSLRSDDTAVYYCAR **GGLPGDFDY**WGQGTTVTVSS ASTKGPSVFPLAPSSKSTSGGTAALGCLVKDYFPEPVTVSWNSGALTSG VHTFPAVLQSSGLYSLSSVVTVPSSSLGTQTYICNVNHKPSNTKVDKKV EPKSCDKTHTCPPCPAPELLGGPSVFLFPPKPKDTLMISRTPEVTCVVV DVSHEDPEVKFNWYVDGVEVHNAKTKPREEQYNSTYRVVSVLTVLHQDW LNGKEYKCKVSNKALPAPIEKTISKAKGQPREPQVYTLPPSREEMTKNQ VSLTCLVKGFYPSDIAVEWESNGQPENNYKTTPPVLDSDGSFFLYSKLT VDKSRWQQGNVFSCSVMHEALHNHYTQKSLSLSPGK | 92 |
| 310P3C5-z10 Light chain | DIQMTQSPSSVSASVGDRVTITC**KASEDIYNRLA**WYQQKPGKAPKLLIS **GVTSLET**GVPSRFSGSGSGKDFTLTISSLQPEDFATYYC**QQYWSFPWT**F GGGTKVEIK RTVAAPSVFIFPPSDEQLKSGTASVVCLLNNFYPREAKVQWKVDNALQS GNSQESVTEQDSKDSTYSLSSTLTLSKADYEKHKVYACEVTHQGLSSPV TKSFNRGEC | 93 |
| 310P3C5-z11 Heavy chain | QVQLVQSGAEVKKPGASVKVSCKASGYTFT**DFYMN**WVRQAPGQGLEWIG **DANPNNGGTTHNPRFKG**RATLTVDTSTSTAYMELRSLRSDDTAVYYCAR **GGLPGDFDY**WGQGTTVTVSS ASTKGPSVFPLAPSSKSTSGGTAALGCLVKDYFPEPVTVSWNSGALTSG VHTFPAVLQSSGLYSLSSVVTVPSSSLGTQTYICNVNHKPSNTKVDKKV EPKSCDKTHTCPPCPAPELLGGPSVFLFPPKPKDTLMISRTPEVTCVVV DVSHEDPEVKFNWYVDGVEVHNAKTKPREEQYNSTYRVVSVLTVLHQDW LNGKEYKCKVSNKALPAPIEKTISKAKGQPREPQVYTLPPSREEMTKNQ VSLTCLVKGFYPSDIAVEWESNGQPENNYKTTPPVLDSDGSFFLYSKLT VDKSRWQQGNVFSCSVMHEALHNHYTQKSLSLSPGK | 94 |
| 310P3C5-z11 Light chain | DIQMTQSPSSVSASVGDRVTITC**KASEDIYNRLA**WYQQKPGKAPKLLIS **GVTSLET**GVPSRFSGSGSGKDFTLTISSLQPEDFATYYC**QQYWSFPWT**F GGGTKVEIK RTVAAPSVFIFPPSDEQLKSGTASVVCLLNNFYPREAKVQWKVDNALQS GNSQESVTEQDSKDSTYSLSSTLTLSKADYEKHKVYACEVTHQGLSSPV TKSFNRGEC | 95 |
| 310P3C5-z12 Heavy chain | QVQLVQSGAEVKKPGASVKVSCKASGYTFT**DFYMN**WVRQAPGQGLEWIG **DANPNNGGTTHNPRFKG**QATLTVDKSTSTAYMELRSLRSDDTAVYYCAR **GGLPGDFDY**WGQGTTVTVSS ASTKGPSVFPLAPSSKSTSGGTAALGCLVKDYFPEPVTVSWNSGALTSG VHTFPAVLQSSGLYSLSSVVTVPSSSLGTQTYICNVNHKPSNTKVDKKV EPKSCDKTHTCPPCPAPELLGGPSVFLFPPKPKDTLMISRTPEVTCVVV DVSHEDPEVKFNWYVDGVEVHNAKTKPREEQYNSTYRVVSVLTVLHQDW LNGKEYKCKVSNKALPAPIEKTISKAKGQPREPQVYTLPPSREEMTKNQ VSLTCLVKGFYPSDIAVEWESNGQPENNYKTTPPVLDSDGSFFLYSKLT VDKSRWQQGNVFSCSVMHEALHNHYTQKSLSLSPGK | 96 |
| 310P3C5-z12 Light chain | DIQMTQSPSSVSASVGDRVTITC**KASEDIYNRLA**WYQQKPGKAPKLLIS **GVTSLET**GVPSRFSGSGSGKDFTLTISSLQPEDFATYYC**QQYWSFPWT**F GGGTKVEIK RTVAAPSVFIFPPSDEQLKSGTASVVCLLNNFYPREAKVQWKVDNALQS GNSQESVTEQDSKDSTYSLSSTLTLSKADYEKHKVYACEVTHQGLSSPV TKSFNRGEC | 97 |

(continued)

| 310P3C5 | | |
|---|---|---|
| | **Sequence** | **SEQ NO ID:** |
| 310P3C5-z13 Heavy chain | QVQLVQSGAEVKKPGASVKVSCKASGYTFT**DFYMN**WVRQAPGQGLEWMG **DANPNNGGTTHNPRFKG**RVTMTTDTSTSTAYMELRSLRSDDTAVYYCAR **GGLPGDFDY**WGQGTTVTVSS ASTKGPSVFPLAPSSKSTSGGTAALGCLVKDYFPEPVTVSWNSGALTSG VHTFPAVLQSSGLYSLSSVVTVPSSSLGTQTYICNVNHKPSNTKVDKKV EPKSCDKTHTCPPCPAPELLGGPSVFLFPPKPKDTLMISRTPEVTCVVV DVSHEDPEVKFNWYVDGVEVHNAKTKPREEQYNSTYRVVSVLTVLHQDW LNGKEYKCKVSNKALPAPIEKTISKAKGQPREPQVYTLPPSREEMTKNQ VSLTCLVKGFYPSDIAVEWESNGQPENNYKTTPPVLDSDGSFFLYSKLT VDKSRWQQGNVFSCSVMHEALHNHYTQKSLSLSPGK | 98 |
| 310P3C5-z13 Light chain | DIQMTQSPSSLSASVGDRVTITC**KASEDIYNRLA**WYQQKPGKAPKLLIS **GVTSLET**GVPSRFSGSGSGTDFTFTISSLQPEDIATYYC**QQYWSFPWT**F GGGTKVEIK RTVAAPSVFIFPPSDEQLKSGTASVVCLLNNFYPREAKVQWKVDNALQS GNSQESVTEQDSKDSTYSLSSTLTLSKADYEKHKVYACEVTHQGLSSPV TKSFNRGEC | 99 |
| 310P3C5-z14 Heavy chain | QVQLVQSGAEVKKPGASVKVSCKASGYTFT**DFYMN**WVKQAPGQGLEWIG **DANPNNGGTTHNPRFKG**RATLTVDKSASTAYMELRSLRSEDTAVYYCAR **GGLPGDFDY**WGQGTTVTVSS ASTKGPSVFPLAPSSKSTSGGTAALGCLVKDYFPEPVTVSWNSGALTSG VHTFPAVLQSSGLYSLSSVVTVPSSSLGTQTYICNVNHKPSNTKVDKKV EPKSCDKTHTCPPCPAPELLGGPSVFLFPPKPKDTLMISRTPEVTCVVV DVSHEDPEVKFNWYVDGVEVHNAKTKPREEQYNSTYRVVSVLTVLHQDW LNGKEYKCKVSNKALPAPIEKTISKAKGQPREPQVYTLPPSREEMTKNQ VSLTCLVKGFYPSDIAVEWESNGQPENNYKTTPPVLDSDGSFFLYSKLT VDKSRWQQGNVFSCSVMHEALHNHYTQKSLSLSPGK | 100 |
| 310P3C5-z14 Light chain | DIQMTQSPSSVSASVGDRVTITC**KASEDIYNRLA**WYQQKPGKAPKLLIY **GVTSLET**GVPSRFSGSGSGTDFTLTISSLQPEDFATYYC**QQYWSFPWT**F GGGTKVEIK RTVAAPSVFIFPPSDEQLKSGTASVVCLLNNFYPREAKVQWKVDNALQS GNSQESVTEQDSKDSTYSLSSTLTLSKADYEKHKVYACEVTHQGLSSPV TKSFNRGEC | 101 |
| 310P3C5-z15 Heavy chain | QVQLVQSGAEVKKPGASVKVSCKASGYTFT**DFYMN**WVKQAPGQGLEWIG **DANPNNGGTTHNPRFKG**RATLTVDKSASTAYMELRSLRSEDTAVYYCAR **GGLPGDFDY**WGQGTTVTVSS ASTKGPSVFPLAPSSKSTSGGTAALGCLVKDYFPEPVTVSWNSGALTSG VHTFPAVLQSSGLYSLSSVVTVPSSSLGTQTYICNVNHKPSNTKVDKKV EPKSCDKTHTCPPCPAPELLGGPSVFLFPPKPKDTLMISRTPEVTCVVV DVSHEDPEVKFNWYVDGVEVHNAKTKPREEQYNSTYRVVSVLTVLHQDW LNGKEYKCKVSNKALPAPIEKTISKAKGQPREPQVYTLPPSREEMTKNQ VSLTCLVKGFYPSDIAVEWESNGQPENNYKTTPPVLDSDGSFFLYSKLT VDKSRWQQGNVFSCSVMHEALHNHYTQKSLSLSPGK | 102 |
| 310P3C5-z15 Light chain | DIQMTQSPSSLSASVGDRVTITC**KASEDIYNRLA**WYQQKPGKAPKLLIS **GVTSLET**GVPSRFSGSGSGTDFTFTISSLQPEDIATYYC**QQYWSFPWT**F GGGTKVEIK RTVAAPSVFIFPPSDEQLKSGTASVVCLLNNFYPREAKVQWKVDNALQS GNSQESVTEQDSKDSTYSLSSTLTLSKADYEKHKVYACEVTHQGLSSPV TKSFNRGEC | 103 |

**Example 5. Antigen binding properties of the humanized antibodies**

### *5.1 Binding to recombinant human DLL3*

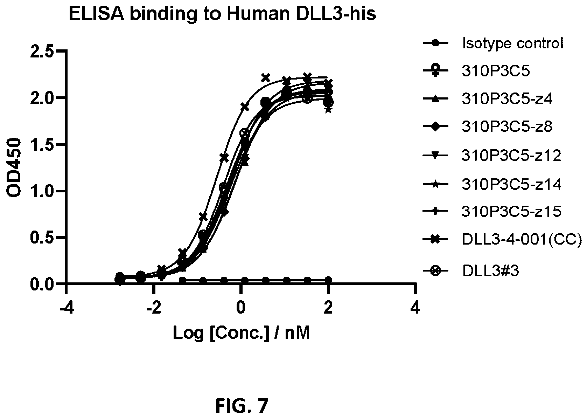

To evaluate the antigen binding activity, the humanized antibodies were subjected to ELISA tested as described before. As shown in **FIG. 7,** all the humanized 310P3C5 antibodies showed comparable binding efficacy to human DLL3 protein with their parental chimeric antibodies.

### *5.2 Affinity ranking of humanized antibodies by Biacore™*

To explore whether the humanized antibodies could maintain their binding kinetics, single dose affinity ranking were performed with Biacore ™. The antibodies (2 ug/ml) were captured with Protein A chips. Human DLL3-his protein at 100nM was injected over captured antibodies for 120 s at a flow rate of 30 μL/min. The antigen was allowed to dissociate for 400 s. The experiment was carried out on a Biacore ™ 8K. Data analysis was carried out using Biacore ™ 8K evaluation software.

The results shown in **Table 9** demonstrated that several humanized antibodies including 310P3C5-z4, 310P3C5-z8, 310P3C5-z12, 310P3C5-z14 and 310P3C5-z15 showed comparable affinity to their chimeric antibodies.

*Table 9. Affinity ranking results of humanized antibodies*

| Affinity / Antibody | Human DLL3 His | | |
|---|---|---|---|
| | ka(1/Ms) | Kd(1/s) | KD(M) |
| 310P3C5 | 9.71E+05 | 3.96E-04 | 4.08E-10 |
| 310P3C5-z1 | 7.14E+05 | 1.46E-03 | 2.05E-09 |
| 310P3C5-z2 | 8.01E+05 | 1.20E-03 | 1.50E-09 |
| 310P3C5-z3 | 5.56E+05 | 1.21E-03 | 2.18E-09 |
| 310P3C5-z4 | 8.40E+05 | 2.79E-04 | 3.32E-10 |
| 310P3C5-z5 | 8.75E+05 | 1.83E-03 | 2.09E-09 |
| 310P3C5-z6 | 1.00E+06 | 1.43E-03 | 1.42E-09 |
| 310P3C5-z7 | 7.78E+05 | 1.47E-03 | 1.89E-09 |
| 310P3C5-z8 | 5.93E+05 | 2.58E-04 | 4.35E-10 |
| 310P3C5-z9 | 8.56E+05 | 1.63E-03 | 1.91E-09 |
| 310P3C5-z10 | 5.95E+05 | 1.01E-03 | 1.70E-09 |
| 310P3C5-z11 | 6.25E+05 | 1.15E-03 | 1.84E-09 |
| 310P3C5-z12 | 9.53E+05 | 2.78E-04 | 2.91E-10 |
| 310P3C5-z14 | 8.53E+05 | 5.14E-04 | 6.02E-10 |
| 310P3C5-z15 | 8.72E+05 | 7.08E-04 | 8.12E-10 |

### *5.3 Binding to human DLL3 and cynomolgus DLL3 over-expressed HEK293 cells*

To evaluate the binding property to human DLL3 and cynomolgus DLL3 over-expressed on the cells, selected humanized antibodies were analyzed by FACS following the protocols as described in previous examples.

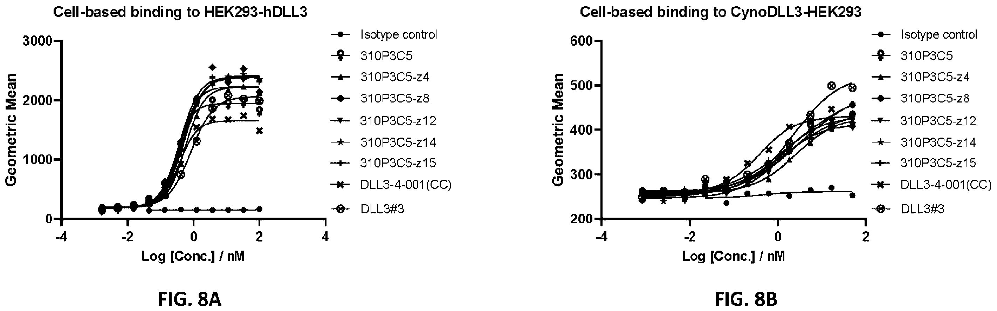

As shown in **FIG. 8A** and **FIG. 8B,** all the selected humanized antibodies including 310P3C5-z4, 310P3C5-z8, 310P3C5-z12, 310P3C5-z14 and 310P3C5-z15 showed maintained binding to human DLL3 over-expressed on HEK293 cells when compared with their chimeric antibodies, which was significantly superior to two benchmark antibodies DLL3-4-001(CC) and DLL3#3. Moreover, all these humanized mAbs maintained binding to cyno DLL3 expressed on HEK293 cells.

### *5.4 Binding to human DLL3 expressed on cancer cell lines*

To evaluate the binding property to human DLL3 expressed on cancer cells, selected humanized antibodies were analyzed by FACS following the protocols as described before.

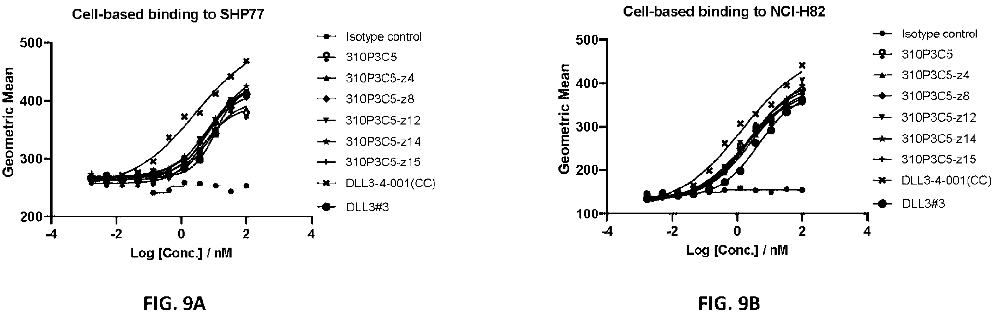

As shown in **FIG. 9A** and **FIG. 9B,** all the selected humanized antibodies including 310P3C5-z4, 310P3C5-z8, 310P3C5-z12, 310P3C5-z14 and 310P3C5-z15 showed maintained binding to human DLL3 endogenously expressed on tumor cells SHP77 and NCI-H82 when compared with their chimeric antibodies, which was better than the benchmark antibody DLL3#3.

*5.5 Binding to human DLL1 and DLL4 over-expressed on CHO-K1 cells*

To exclude the non-specific binding of the humanized antibody to human DLL1 and DLL4 expressed on cells, selected humanized antibodies were analyzed by FACS following the protocols as described in previous example.

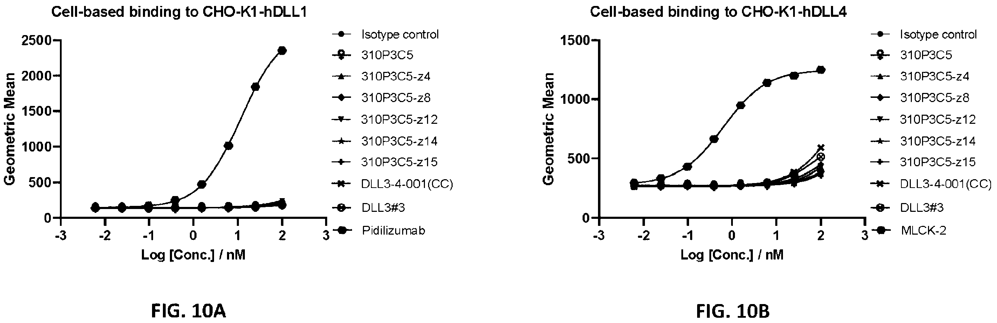

As shown in **FIG. 10A** and **FIG. 10B,** all the humanized antibodies including 310P3C5-z4, 310P3C5-z8, 310P3C5-z12, 310P3C5-z14 and 310P3C5-z15 showed neglectable binding to human DLL1 and DLL4 over-expressed on CHO-K1 cells.

**Example 6. Optimization of the humanized antibodies**

In certain cases, the humanized antibody is further optimized to improve its developability including long-term stability, manufacturability, and low heterogeneity. One of critical factor, post-translational modification (PTM) such as deamidation, isomerization, glycosylation and oxidation, can affect the developability and further compromise the potency, efficacy, and safety of therapeutic antibodies. In this example, computational tools have been used to predict PTM liable sites to facilitate engineering antibodies with better developability.

*6.1 Design of PTM site removal*

In this case, MOE was used to predict PTM liable sites. The amino acid N55 located in the CDR region of the heavy chain of 310P3C5 antibody was identified as a potential deamidation site. Thus, this amino acid was replaced with A, F, H, R, V, W or Y. The sequences of the variable region of the PTM removed humanized antibodies were shown in **Table 10A,** with the modified VH CDR2 sequences shown in **Table 10B.** The variable regions of humanized antibodies were then fused to the constant region of human IgG1 for antibody production and functional characterization.

***Table 10A. The sequences of variable region of PTM removed humanized antibodies (underlining indicates CDR; bold/italic indicates predicted PTM site and mutations)***

| Antibody | Sequence | SEQ NO ID: |
|---|---|---|
| 310P3C5-z15 VH | QVQLVQSGAEVKKPGASVKVSCKASGYTFTDFYMNWVKQAPGQGLEWIGDANPN***N***GGTTHNPRFKGRATLTVDKSASTAYMELRSLRSEDTAVYYCARGGLPGDFDYWGQGTTVTVSS | 69 |
| 310P3C5-z15p10 VH | QVQLVQSGAEVKKPGASVKVSCKASGYTFTDFYMNWVKQAPGQGLEWIGDANPN***A***GGTTHNPRFKGRATLTVDKSASTAYMELRSLRSEDTAVYYCARGGLPGDFDYWGQGTTVTVSS | 104 |
| 310P3C5-z15p12 VH | QVQLVQSGAEVKKPGASVKVSCKASGYTFTDFYMNWVKQAPGQGLEWIGDANPN***F***GGTTHNPRFKGRATLTVDKSASTAYMELRSLRSEDTAVYYCARGGLPGDFDYWGQGTTVTVSS | 105 |
| 310P3C5-z15p13 VH | QVQLVQSGAEVKKPGASVKVSCKASGYTFTDFYMNWVKQAPGQGLEWIGDANPN***H***GGTTHNPRFKGRATLTVDKSASTAYMELRSLRSEDTAVYYCARGGLPGDFDYWGQGTTVTVSS | 106 |
| 310P3C5-z15p18 VH | QVQLVQSGAEVKKPGASVKVSCKASGYTFTDFYMNWVKQAPGQGLEWIGDANPN***R***GGTTHNPRFKGRATLTVDKSASTAYMELRSLRSEDTAVYYCARGGLPGDFDYWGQGTTVTVSS | 107 |
| 310P3C5-z15p19 VH | QVQLVQSGAEVKKPGASVKVSCKASGYTFTDFYMNWVKQAPGQGLEWIGDANPN***V***GGTTHNPRFKGRATLTVDKSASTAYMELRSLRSEDTAVYYCARGGLPGDFDYWGQGTTVTVSS | 108 |
| 310P3C5-z15p20 VH | QVQLVQSGAEVKKPGASVKVSCKASGYTFTDFYMNWVKQAPGQGLEWIGDANPN***W***GGTTHNPRFKGRATLTVDKSASTAYMELRSLRSEDTAVYYCARGGLPGDFDYWGQGTTVTVSS | 109 |
| 310P3C5-z15p21 VH | QVQLVQSGAEVKKPGASVKVSCKASGYTFTDFYMNWVKQAPGQGLEWIGDANPN***Y***GGTTHNPRFKGRATLTVDKSASTAYMELRSLRSEDTAVYYCARGGLPGDFDYWGQGTTVTVSS | 110 |

(continued)

| Antibody | Sequence | SEQ NO ID: |
|---|---|---|
| 310P3C5-z15 VL | DIQMTQSPSSLSASVGDRVTITCKASEDIYNRLAWYQQKPGKAPKLLISGVTSLETGVPSRFSGSGSGTDFTFTISSLQPEDIATYYCQQYWSFPWTFGGGTKVEIK | 73 |

*Table 10B. Optimized VH CDR2 Sequences*

| Antibody | Sequence | SEQ NO ID: |
|---|---|---|
| 310P3C5-z15 VH | DANPN***N***GGTTHNPRFKG | 38 |
| 310P3C5-z15p10 VH | DANPN***A***GGTTHNPRFKG | 111 |
| 310P3C5-z15p12 VH | DANPN***F***GGTTHNPRFKG | 112 |
| 310P3C5-z15p13 VH | DANPN***H***GGTTHNPRFKG | 113 |
| 310P3C5-z15p18 VH | DANPN***R***GGTTHNPRFKG | 114 |
| 310P3C5-z15p19 VH | DANPN***V***GGTTHNPRFKG | 115 |
| 310P3C5-z15p20 VH | DANPN***W***GGTTHNPRFKG | 116 |
| 310P3C5-z15p21 VH | DANPN***Y***GGTTHNPRFKG | 117 |

### *6. 2 Binding of the PTM removed humanized antibodies to human DLL3 expressed HEK293 cells*

To evaluate the binding property to human DLL3 expressed on cells, PTM removed humanized antibodies were analyzed by FACS following the protocols as described in previous examples.

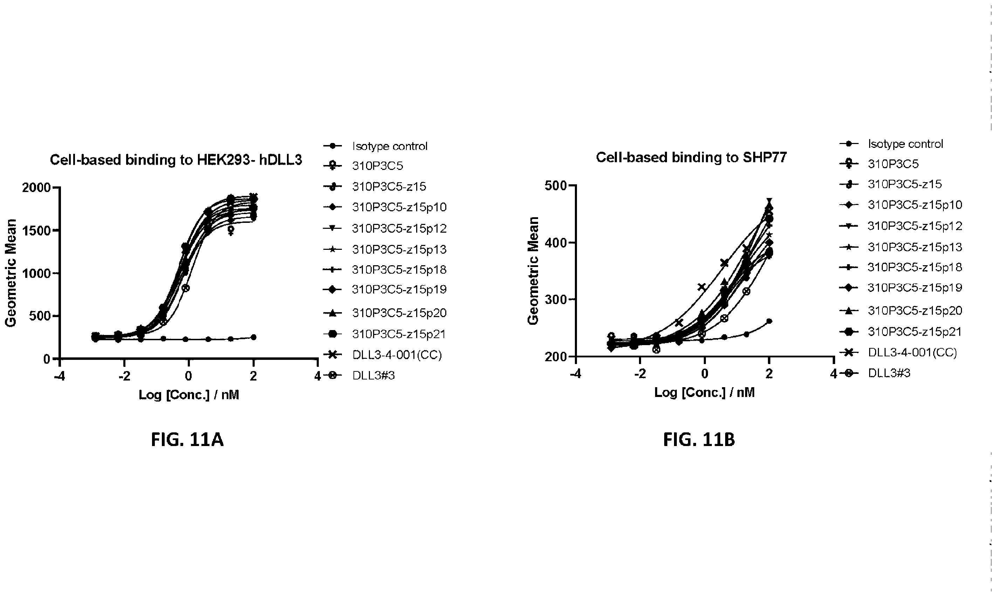

As shown in **FIG. 11A** and **FIG. 11B**, all the selected PTM removed humanized antibodies showed maintained binding to human DLL3 either over-expressed on HEK-293 cells or endogenously expressed in tumor cell line SHP77 cells when compared with their parental humanized antibodies 310P3C5-z15.

The present disclosure is not to be limited in scope by the specific embodiments described which are intended as single illustrations of individual aspects of the disclosure, and any compositions or methods which are functionally equivalent are within the scope of this disclosure. It will be apparent to those skilled in the art that various modifications and variations can be made in the methods and compositions of the present disclosure without departing from the spirit or scope of the disclosure. Thus, it is intended that the present disclosure cover the modifications and variations of this disclosure provided they come within the scope of the appended claims and their equivalents.

All publications and patent applications mentioned in this specification are herein incorporated by reference to the same extent as if each individual publication or patent application was specifically and individually indicated to be incorporated by reference.

## Claims

1. An antibody or antigen-binding fragment thereof which has specificity to the human delta-like ligand 3 (DLL3) protein and comprises a heavy chain variable region (VH) comprising a VH CDR1, a VH CDR2 and a VH CDR3, and a light chain variable region (VL) comprising a VL CDR1, a VL CDR2, and a VL CDR3, wherein:
   the VH CDR1 comprises the amino acid sequence of SEQ ID NO: 37;

   the VH CDR2 comprises an amino acid sequence selected from the group consisting of SEQ ID NO: 38 and 111-117;
   the VH CDR3 comprises the amino acid sequence of SEQ ID NO: 39;
   the VL CDR1 comprises the amino acid sequence of SEQ ID NO: 40;
   the VL CDR2 comprises the amino acid sequence of SEQ ID NO: 41; and
   the VL CDR3 comprises the amino acid sequence of SEQ ID NO: 42.

2. The antibody or antigen-binding fragment thereof of claim 1, wherein the VH comprises an amino acid sequence selected from the group consisting of SEQ ID NO: 9, 65-69 and 104-110 or a peptide having at least 90% sequence identity to an amino acid sequence selected from the group consisting of SEQ ID NO: 9, 65-69 and 104-110.

**3.** The antibody or antigen-binding fragment thereof of claim 1 or 2, wherein the VL comprises an amino acid sequence selected from the group consisting of SEQ ID NO: 10 and 70-73 or a peptide having at least 90% sequence identity to an amino acid sequence selected from the group consisting of SEQ ID NO: 10 and 70-73.

**4.** The antibody or antigen-binding fragment thereof of claim 3, wherein the VH comprises an amino acid sequence selected from the group consisting of SEQ ID NO: 104-110 and the VL comprises the amino acid sequence of SEQ ID NO: 73.

**5.** An antibody or antigen-binding fragment thereof which has specificity to the human delta-like ligand 3 (DLL3) protein, which competes with the antibody or antigen-binding fragment thereof of any one of claims 1-4 in binding to the DLL3 protein.

**6.** The antibody or antigen-binding fragment thereof of any one of claims 1-5, wherein the antibody or fragment thereof is a bivalent Fab antibody, or a fragment selected from the group consisting of F(ab')2, F(ab)2, Fab', Fab, Fv, and scFv, optionally the antibody or antigen-binding fragment thereof is humanized.

**7.** A multispecific antibody comprising the antibody or antigen-binding fragment of any one of claims 1-6 and one or more antibodies or antigen-binding fragments thereof having binding specificity to a target antigen that is not DLL3.

**8.** A chimeric antigen receptor (CAR) comprising the antibody or antigen-binding fragment thereof of any one of claims 1-6, a transmembrane domain, a costimulatory domain, and a CD3ξ intracellular domain.

**9.** One or more polynucleotide(s) encoding the antibody or antigen-binding fragment thereof of any one of claims 1-7 or the CAR of claim 8.

**10.** The polynucleotide(s) of claim 9, which is one or more mRNA.

**11.** The polynucleotide(s) of claim 10, wherein the mRNA is chemically modified.

**12.** A cell comprising the polynucleotide(s) of claim 9 to 11.

**13.** A composition comprising the antibody or antigen-binding fragment thereof of any one of claims 1-7 or the CAR of claim 8, and a pharmaceutically acceptable carrier.

**14.** The antibody or antigen-binding fragment thereof of any one of claims 1-7 or the CAR of claim 8, or the composition of claim 13 for use in treating cancer.

**15.** The antibody or antigen-binding fragment thereof, the CAR, or the composition for use of claim 14, wherein the cancer is selected from the group consisting of ovarian cancer, prostate cancer, cancer of the urinary tract, pancreatic cancer, lung cancer, breast cancer, bladder cancer, colorectal cancer, endometrial cancer, esophageal cancer, head and neck cancer, kidney cancer, leukemia, liver cancer, lymphoma, melanoma, and thyroid cancer, optionally wherein the cancer is small cell lung cancer (SCLC).

ELISA binding to Human DLL3-his
OD450
Log [Conc.] / nM
Isotype control
9E8D8
36B7F3
129H2B9
148C3A7
310P3C5
362H3D3
DLL3-4-001(CC)
DLL3#3
ELISA binding to Cyno DLL3-his
OD450
Log [Conc.] / nM
Isotype control
9E8D8
36B7F3
129H2B9
148C3A7
310P3C5
362H3D3
DLL3-4-001(CC)
DLL3#3
ELISA binding to Mouse DLL3-his
OD450
Log [Conc.] / nM
Isotype control
9E8D8
36B7F3
129H2B9
148C3A7
310P3C5
362H3D3
DLL3-4-001(CC)
DLL3#3

FIG. 1A

FIG. 1B

FIG. 1C

ELISA binding to Human DLL1-his
OD450
0.6
0.4
0.2
0.0
-3
-2
-1
0
1
2
3
Log [Conc.] / nM
Isotype control
9E8D8
36B7F3
129H2B9
148C3A7
310P3C5
362H3D3
DLL3-4-001(CC)
DLL3#3
Pidilizumab

FIG. 2A

ELISA binding to Human DLL4-his
OD450
2.0
1.5
1.0
0.5
0.0
-3
-2
-1
0
1
2
3
Log [Conc.] / nM
Isotype control
9E8D8
36B7F3
129H2B9
148C3A7
310P3C5
362H3D3
DLL3-4-001(CC)
DLL3#3
MLCK-2

FIG. 2B

Domain Mapping
MFI (Geometric Mean)
15000
10000
5000
0
EGF1+2
EGF2+3
EGF3+4
EGF4+5
EGF5+6
EGF6+MPER
Isotyoe Control
9E8D8
36B7F3
129H2B9
148C3A7
310P3C5
362H3D3
DLL3-4-001(CC)
DLL3#3

FIG. 3

Cell-based binding to HEK293-hDLL3
Geometric Mean
4000
3000
2000
1000
0
-4
-2
0
2
Log [Conc.] / nM
Isotype control
9E8D8
36B7F3
129H2B9
148C3A7
310P3C5
362H3D3
DLL3-4-001(CC)
DLL3#3
Cell-based binding to HEK293-CynoDLL3
Geometric Mean
3000
2000
1000
0
-4
-2
0
2
Log [Conc.] / nM
Isotype control
9E8D8
36B7F3
129H2B9
148C3A7
310P3C5
362H3D3
DLL3-4-001(CC)
DLL3#3


FIG. 4A

FIG. 4B

Cell-based binding to SHP77
Geometric Mean
500
400
300
200
-4
-2
0
2
4
Log [Conc.] / nM
Isotype control
9E8D8
36B7F3
129H2B9
148C3A7
310P3C5
362H3D3
DLL3-4-001(CC)
DLL3#3
Cell-based binding to NCI-H82
Geometric Mean
500
400
300
200
100
0
-4
-2
0
2
4
Log [Conc.] / nM
Isotype control
9E8D8
36B7F3
129H2B9
148C3A7
310P3C5
362H3D3
DLL3-4-001(CC)
DLL3#3


FIG. 5A

FIG. 5B

Cell-based binding to CHO-K1-hDLL1
Geometric Mean
2500
2000
1500
1000
500
0
-3
-2
-1
0
1
2
3
Log [Conc.] / nM
Isotype control
9E8D8
36B7F3
129H2B9
148C3A7
310P3C5
362H3D3
DLL3-4-001(CC)
DLL3#3
Pidilizumab
Cell-based binding to CHO-K1-hDLL4
Geometric Mean
1000
800
600
400
200
0
-3
-2
-1
0
1
2
3
Log [Conc.] / nM
Isotype control
9E8D8
36B7F3
129H2B9
148C3A7
310P3C5
362H3D3
DLL3-4-001(CC)
DLL3#3
MLCK-2

FIG. 6A

FIG. 6B

ELISA binding to Human DLL3-his
OD450
2.5
2.0
1.5
1.0
0.5
0.0
-4
-2
0
2
4
Log [Conc.] / nM
Isotype control
310P3C5
310P3C5-z4
310P3C5-z8
310P3C5-z12
310P3C5-z14
310P3C5-z15
DLL3-4-001(CC)
DLL3#3

FIG. 7

Cell-based binding to CynoDLL3-HEK293
Geometric Mean
600
500
400
300
200
-4
-2
0
2
Log [Conc.] / nM
Isotype control
310P3C5
310P3C5-z4
310P3C5-z8
310P3C5-z12
310P3C5-z14
310P3C5-z15
DLL3-4-001(CC)
DLL3#3

FIG. 8B

Cell-based binding to HEK293-hDLL3
Geometric Mean
3000
2000
1000
0
-4
-2
0
2
4
Log [Conc.] / nM
Isotype control
310P3C5
310P3C5-z4
310P3C5-z8
310P3C5-z12
310P3C5-z14
310P3C5-z15
DLL3-4-001(CC)
DLL3#3

FIG. 8A

Cell-based binding to SHP77
Geometric Mean
500
400
300
200
-4
-2
0
2
4
Log [Conc.] / nM
Isotype control
310P3C5
310P3C5-z4
310P3C5-z8
310P3C5-z12
310P3C5-z14
310P3C5-z15
DLL3-4-001(CC)
DLL3#3

FIG. 9A

Cell-based binding to NCI-H82
Geometric Mean
500
400
300
200
100
-4
-2
0
2
4
Log [Conc.] / nM
Isotype control
310P3C5
310P3C5-z4
310P3C5-z8
310P3C5-z12
310P3C5-z14
310P3C5-z15
DLL3-4-001(CC)
DLL3#3

FIG. 9B

Cell-based binding to CHO-K1-hDLL1
Geometric Mean
2500
2000
1500
1000
500
0
-3
-2
-1
0
1
2
3
Log [Conc.] / nM
Isotype control
310P3C5
310P3C5-z4
310P3C5-z8
310P3C5-z12
310P3C5-z14
310P3C5-z15
DLL3-4-001(CC)
DLL3#3
Pidilizumab
Cell-based binding to CHO-K1-hDLL4
Geometric Mean
1500
1000
500
0
-3
-2
-1
0
1
2
3
Log [Conc.] / nM
Isotype control
310P3C5
310P3C5-z4
310P3C5-z8
310P3C5-z12
310P3C5-z14
310P3C5-z15
DLL3-4-001(CC)
DLL3#3
MLCK-2


FIG. 10A

FIG. 10B

Cell-based binding to HEK293- hDLL3
Geometric Mean
2000
1500
1000
500
0
-4
-2
0
2
4
Log [Conc.] / nM
Isotype control
310P3C5
310P3C5-z15
310P3C5-z15p10
310P3C5-z15p12
310P3C5-z15p13
310P3C5-z15p18
310P3C5-z15p19
310P3C5-z15p20
310P3C5-z15p21
DLL3-4-001(CC)
DLL3#3
Cell-based binding to SHP77
Geometric Mean
500
400
300
200
-4
-2
0
2
4
Log [Conc.] / nM
Isotype control
310P3C5
310P3C5-z15
310P3C5-z15p10
310P3C5-z15p12
310P3C5-z15p13
310P3C5-z15p18
310P3C5-z15p19
310P3C5-z15p20
310P3C5-z15p21
DLL3-4-001(CC)
DLL3#3

FIG. 11A

FIG. 11B

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2005035572 A
- WO 03035694 A
- WO 0029004 A
- WO 02051870 A
- US 5892019 A
- US 4373071 A
- US 4401796 A
- US 4415732 A
- US 4458066 A
- US 4500707 A
- US 4668777 A
- US 4973679 A
- US 5047524 A
- US 5132418 A
- US 5153319 A
- US 5262530 A
- US 5700642 A
- US 6150584 A
- US 6458592 B
- US 6420140 B
- WO 2019234220 A
- WO 2015005632 A


### Non-patent literature cited in the description


- *Nature*, 1989, vol. 341, 544-546
- *Dev Comp Immunol*, 2006, vol. 30, 43-56
- *Trend Biochem Sci*, 2001, vol. 26, 230-235
- *Trends Biotechnol*, 2003, vol. 21, 484-490
- *Febs Lett*, 1994, vol. 339, 285-290
- **HAMERS-CASTERMAN et al.** *Nature*, 1993, vol. 363, 446-448
- **KABAT, E et al.** Sequences of Proteins of Immunological Interest. U.S. Department of Health and Human Services, 1983
- **CHOTHIA** ; **LESK**. *J. Mol. Biol.*, 1987, vol. 196, 901-917
- **KABAT et al.** Sequences of Proteins of Immunological Interest. *U.S. Dept. of Health and Human Services*, 1983
- **CHOTHIA et al.** *J. Mol. Biol.*, 1987, vol. 196, 901-917
- **KABAT et al.** Sequence of Proteins of Immunological Interest. *U.S. Dept. of Health and Human Services*, 1983
- **BURTON**. *Molec. Immunol.*, 1985, vol. 22, 161-206
- **ROUX et al.** *J. Immunol*, 1998, vol. 161, 4083
- *CHEMICAL ABSTRACTS*, 1036730-42-3